# EUROPEAN PATENT APPLICATION

(11) **EP 4 349 986 A1**
(43) Date of publication of application: **10.04.2024**
(21) Application number: 22811320.5
(22) Date of filing: 24.05.2022
(51) Int. Cl.: C12N 15/113, A61K 9/08, A61K 31/713, A61P 25/00, A61P 29/00, A61P 43/00

(54) **HETERONUCLEIC ACID CONTAINING SCPBNA OR AMNA**

(30) Priority: 25.05.2021 JP 2021087941
(71) Applicant: National University Corporation Tokyo Medical and Dental University, Tokyo 113-8510 (JP); OSAKA UNIVERSITY, Suita-shi Osaka 565-0871 (JP)
(72) Inventor: YOKOTA Takanori, Tokyo 113-8510 (JP); NAGATA Tetsuya, Tokyo 113-8510 (JP); YOSHIOKA Kotaro, Tokyo 113-8510 (JP); OBIKA Satoshi, Suita-shi, Osaka 565-0871 (JP)
(74) Representative: Mewburn Ellis LLP
(86) International application number: PCT/JP2022/021251
(87) International publication number: WO 2022/250050

(57) **Abstract**

A problem to be solved by the present invention is to provide a double-stranded nucleic acid complex having a reduced toxicity whose effectiveness is not impaired. Provided is a double-stranded nucleic acid complex comprising a first nucleic acid strand and a second nucleic acid strand, wherein: said first nucleic acid strand is capable of hybridizing to at least part of a target gene or a transcription product thereof, and has an antisense effect on said target gene or transcription product thereof, said second nucleic acid strand comprises a base sequence complementary to said first nucleic acid strand, and said first nucleic acid strand and/or said second nucleic acid strand comprises at least one bridged non-natural nucleoside represented by formula (I) or formula (II) (wherein R represents a hydrogen atom or a methyl group).

## Description

### Technical Field

The present invention relates to a double-stranded nucleic acid complex containing scpBNA or AmNA, a pharmaceutical composition containing the same, and the like.

### Background Art

Recently, in the ongoing development of pharmaceuticals called nucleic acid medicines, oligonucleotides have drawn attention. In particular, the development of a nucleic acid medicine using the antisense method is actively carried out in consideration of the high selectivity of a target gene and low toxicity. The antisense method is a method which comprises selectively modifying or inhibiting the expression of a protein encoded by a target gene or the activity of miRNA by introducing an oligonucleotide (antisense oligonucleotide: sometimes referred herein to as "ASO") complementary to a partial sequence of mRNA or miRNA transcribed from the target gene as a target sense strand.

As a nucleic acid using the antisense method, the present inventors have developed a double-stranded nucleic acid complex (heteroduplex oligonucleotide (HDO)) by annealing an antisense oligonucleotide and a complementary strand thereof (Patent Literature 1, and Non-Patent Literatures 1 and 2).

The double-stranded nucleic acid complex has a high antisense effect and is a revolutionary technique that makes it possible to control the central nervous system beyond the blood-brain barrier. However, since high-dose administration is required for treating a neurological disease such as Alzheimer's disease, a side effect such as serious liver disorder can be problematic.

Thus, a technique that reduces its toxicity without impairing the effectiveness of the double-stranded nucleic acid complex is required.

### Citation List

### Patent Literature

Patent Literature 1: International Publication No. WO2013/089283
Patent Literature 2: International Publication No. WO2014/192310

### Non-Patent Literature

Non-Patent Literature 1: Nishina K, et.al., "DNA/RNA heteroduplex oligonucleotide for highly efficient gene silencing", Nature Communication, 2015, 6: 7969.
Non-Patent Literature 2: Asami Y, et al., "Drug delivery system of therapeutic oligonucleotides", Drug Discoveries & Therapeutics. 2016; 10 (5): 256-262.

### Summary of Invention

### Technical Problem

A problem to be solved by the present invention is to provide a double-stranded nucleic acid complex having a reduced toxicity whose effectiveness is not impaired.

### Solution to Problem

The present inventors conducted intensive studies for solving the problem described above, and introduced 2'-O,4'-C-spirocyclopropylene-bridged nucleic acid (scpBNA) or amido-bridged nucleic acid (AmNA) into a double-stranded nucleic acid complex. As a result, it was found that the toxicity of a double-stranded nucleic acid complex can be dramatically reduced or eliminated by the introduction of scpBNA or AmNA. The toxicity reduction effect is a remarkable effect which is beyond what was previously expected. Furthermore, it was found that the effectiveness of a double-stranded nucleic acid complex is not impaired by the introduction of scpBNA or AmNA. The present invention is based on the findings described above and provides the followings:

(1) A double-stranded nucleic acid complex comprising a first nucleic acid strand and a second nucleic acid strand, wherein: said first nucleic acid strand is capable of hybridizing to at least part of a target gene or a transcription product thereof, and has an antisense effect on said target gene or transcription product thereof, said second nucleic acid strand comprises a base sequence complementary to said first nucleic acid strand, and said first nucleic acid strand and/or said second nucleic acid strand comprises at least one bridged non-natural nucleoside represented by the following formula (I) or formula (II): 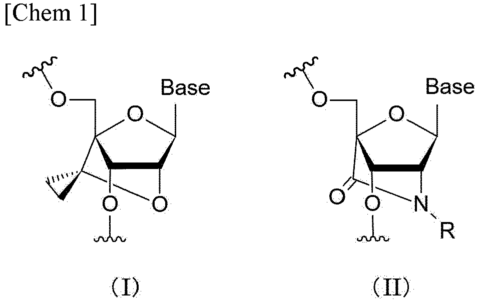 wherein R represents a hydrogen atom or a methyl group.
(2) The double-stranded nucleic acid complex of (1), wherein said first nucleic acid strand is a gapmer.
(3) The double-stranded nucleic acid complex of (2), wherein said first nucleic acid strand comprises:
   [1] a central region comprising at least four consecutive deoxyribonucleosides,
   [2] a 5'-wing region comprising a non-natural nucleoside, positioned on the 5' end side of said central region, and
   [3] a 3'-wing region comprising a non-natural nucleoside, positioned on the 3' end side of said central region.
(4) The double-stranded nucleic acid complex of (3), wherein said 5'-wing region and/or said 3'-wing region comprises the bridged non-natural nucleoside represented by said formula (I) or formula (II).
(5) The double-stranded nucleic acid complex of (3) or (4), wherein said second nucleic acid strand comprises at least four consecutive ribonucleosides which are complementary to the at least four consecutive deoxyribonucleosides in said central region in said first nucleic acid strand.
(6) The double-stranded nucleic acid complex of (5), wherein said second nucleic acid strand further comprises at least two consecutive deoxyribonucleosides.
(7) The double-stranded nucleic acid complex of any of (3) to (6), wherein said second nucleic acid strand comprises the bridged non-natural nucleoside represented by said formula (I) or formula (II) in a region which consists of a base sequence complementary to the 5'-wing region and/or the 3'-wing region in said first nucleic acid strand.
(8) The double-stranded nucleic acid complex of (1), wherein said first nucleic acid strand is a mixmer.
(9) The double-stranded nucleic acid complex of any of (1) to (8), wherein said first nucleic acid strand and/or said second nucleic acid strand comprises at least one nucleoside modified at the 2'-position of the ribose selected from the group consisting of 2'-O-methyl modified nucleoside, 2'-O-methoxyethyl modified nucleoside, and 2'-O-[2-(N-methylcarbamoyl)ethyl] modified nucleoside.
(10) The double-stranded nucleic acid complex of any of (1) to (9), wherein all or part of the internucleoside bond in said first nucleic acid strand and/or said second nucleic acid strand are a modified internucleoside bond.
(11) The double-stranded nucleic acid complex of (10), wherein said modified internucleoside bond is a phosphorothioate bond.
(12) The double-stranded nucleic acid complex of any of (1) to (11), wherein said second nucleic acid strand is bound to a tocopherol or an analog thereof, or a cholesterol or an analog thereof.
(13) The double-stranded nucleic acid complex of any of (1) to (11) which is not bound to a ligand.
(14) The double-stranded nucleic acid complex of any one of (1) to (13), wherein said first nucleic acid strand and said second nucleic acid strand are bound via a cleavable or uncleavable linker.
(15) A pharmaceutical composition comprising the double-stranded nucleic acid complex of any of (1) to (14) as an active ingredient.
(16) The pharmaceutical composition of (15) for treating a central nervous system disease in a subject.
(17) The pharmaceutical composition of (15) or (16), wherein the pharmaceutical composition is administered by intraventricular administration or intrathecal administration.
(18) The pharmaceutical composition of (17), wherein 0.1 mg to 200 mg of said double-stranded nucleic acid complex is administered.
(19) The pharmaceutical composition of (15) or (16), wherein the pharmaceutical composition is administered by intravenous administration or subcutaneous administration.
(20) The pharmaceutical composition of (19), wherein said double-stranded nucleic acid complex is administered at 0.1 mg/kg to 100 mg/kg.
(21) The pharmaceutical composition of any of (15) to (20), wherein induction of inflammation or gliosis, or abnormal increase of a cytokine or a chemokine by the pharmaceutical composition is reduced.

The specification incorporates the contents disclosed in Japanese Patent Application No. 2021-087941 based on which the present application claims a priority.

### Advantageous Effect of Invention

According to the present invention, a double-stranded nucleic acid complex having a reduced toxicity whose effectiveness is not impaired is provided.

### Brief Description of Drawings

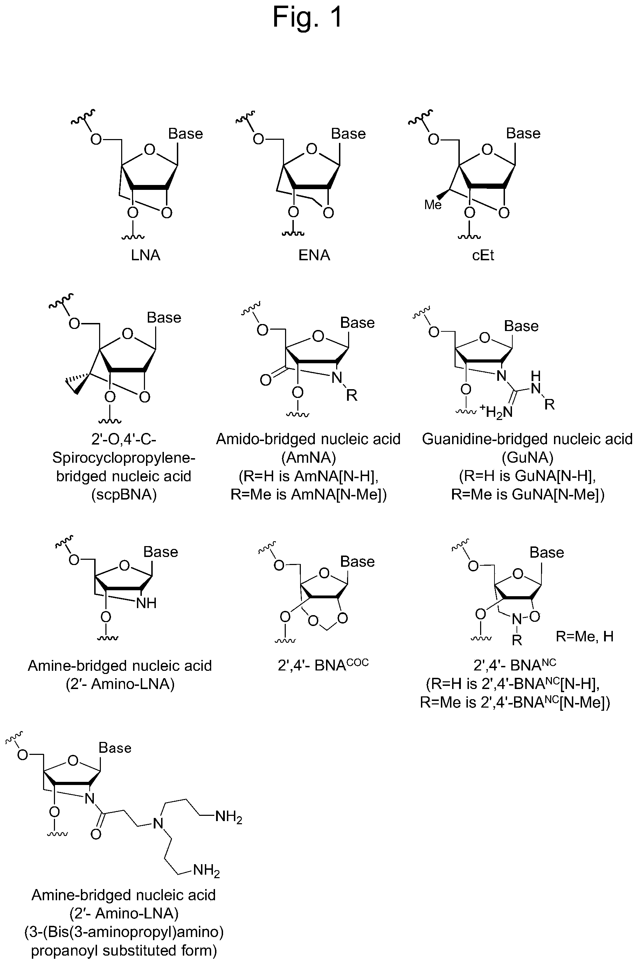

[Figure 1] Figure 1 shows structures of various bridged nucleic acids.

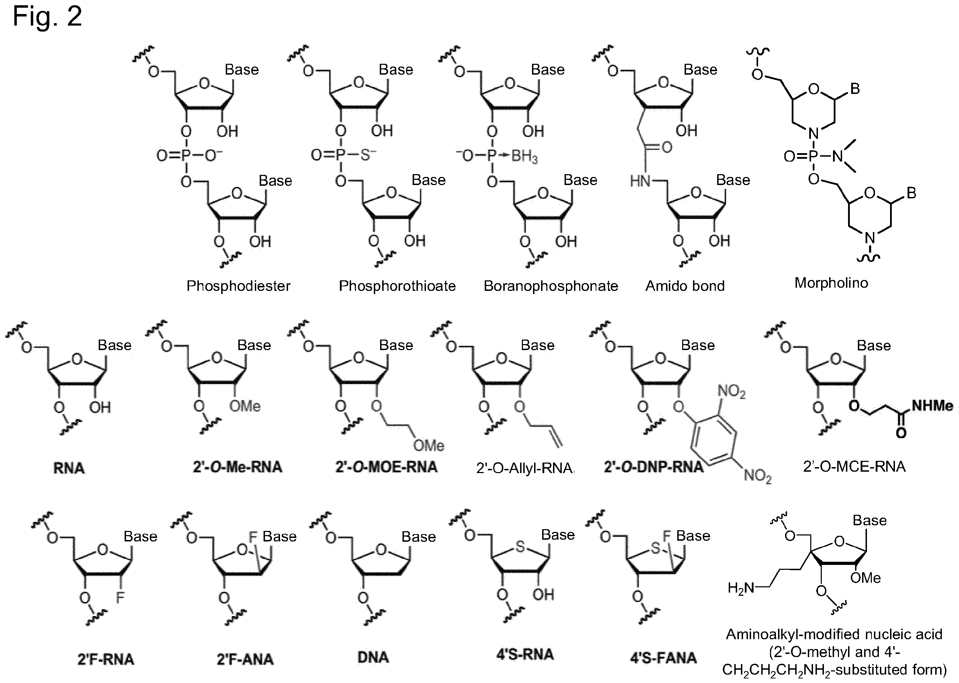

[Figure 2] Figure 2 shows structures of various natural nucleotides or non-natural nucleotides.

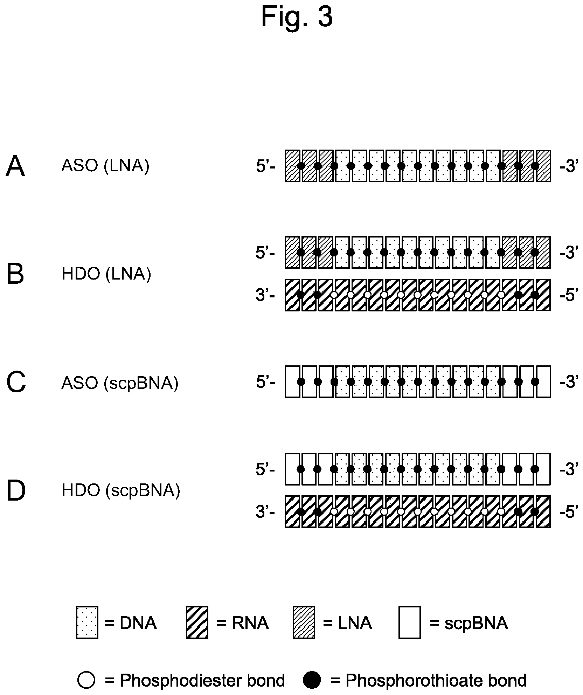

[Figure 3] Figure 3 shows schematic diagrams of nucleic acids used in Example 1. Figure 3A shows the structure of ASO (LNA) containing 3 LNA nucleosides at the 5' end and the 3' end and 10 DNA nucleosides between them. Figure 3B shows the structure of HDO (LNA) containing ASO (LNA) as a first nucleic acid strand and RNA having a complementary sequence to the first nucleic acid strand, as a second nucleic acid strand. Figure 3C shows the structure of ASO (scpBNA) containing 3 scpBNA nucleosides at the 5' end and the 3' end and 10 DNA nucleosides between them. Figure 3D shows the structure of HDO (scpBNA) containing ASO (scpBNA) as a first nucleic acid strand and RNA having a complementary sequence to the first nucleic acid strand, as a second nucleic acid strand.

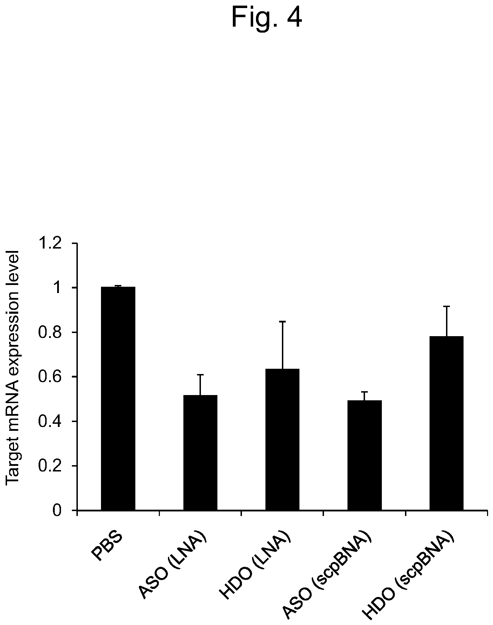

[Figure 4] Figure 4 shows the Mapt mRNA expression levels in the hippocampus of mice to which various nucleic acid agents are administered intraventricularly. The error bars show standard errors.

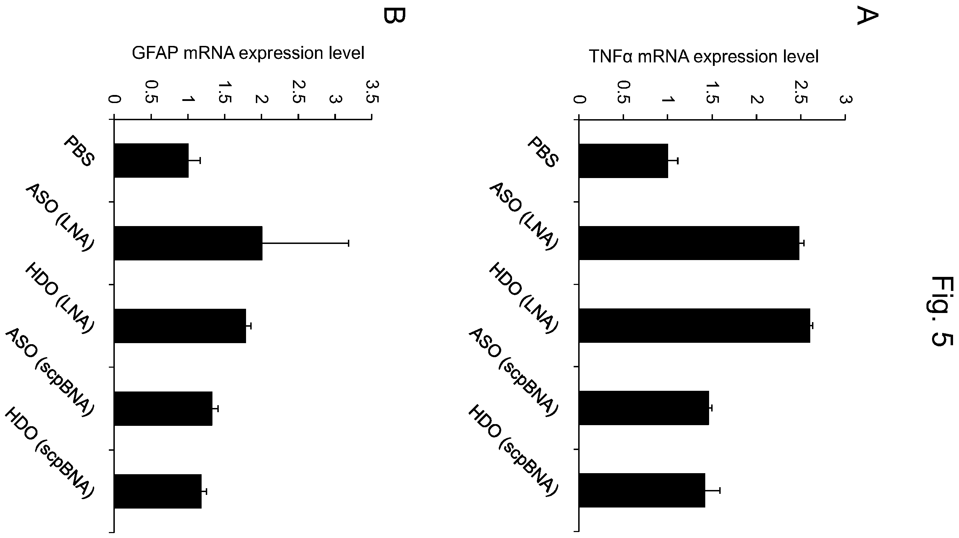

[Figure 5] Figure 5 shows the TNFα mRNA and GFAP mRNA expression levels in the hippocampus of mice to which various nucleic acid agents are administered intraventricularly. Figure 5A shows the expression level of TNFα mRNA. Figure 5B the expression level of GFAP mRNA. The error bars show standard errors.

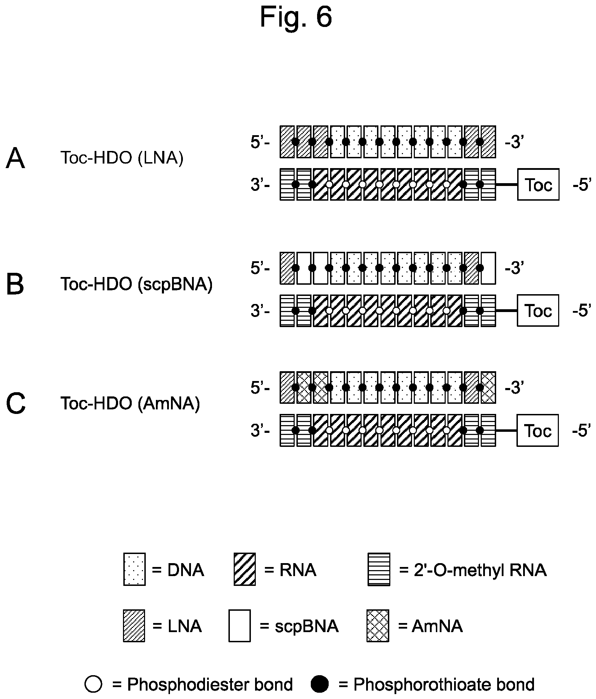

[Figure 6] Figure 6 shows schematic diagrams of nucleic acids used in Example 2. Figure 6A shows the structure of Toc-HDO (LNA). Figure 6B shows the structure of Toc-HDO (scpBNA). Figure 6C shows the structure of Toc-HDO (AmNA). Toc represents tocopherol.

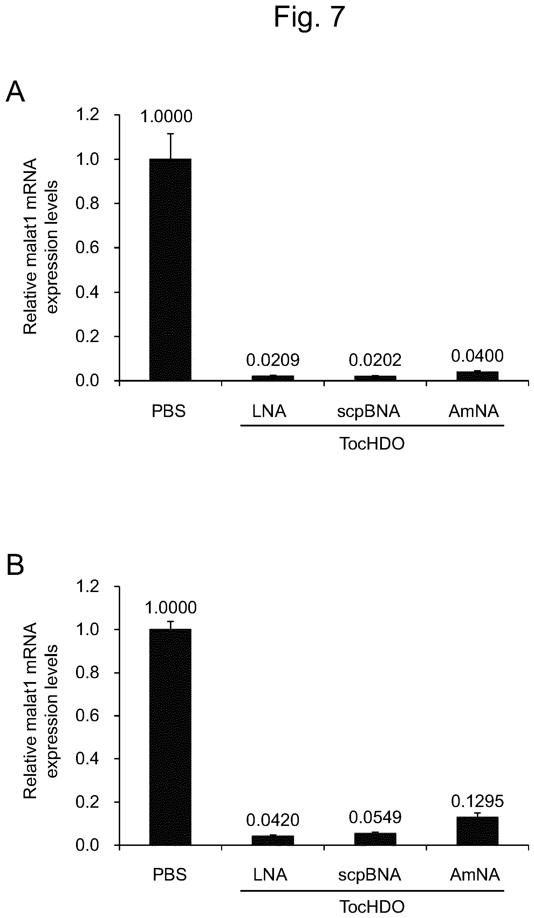

[Figure 7] Figure 7 shows expression levels of malat1 mRNA of mice intravenously administered in a single dose with double-stranded nucleic acid complex agents. Figure 7A shows the expression level of mal at 1 mRNA in the liver. Figure 7B shows the expression level of malat1 mRNA in the kidney. The error bars show standard errors.

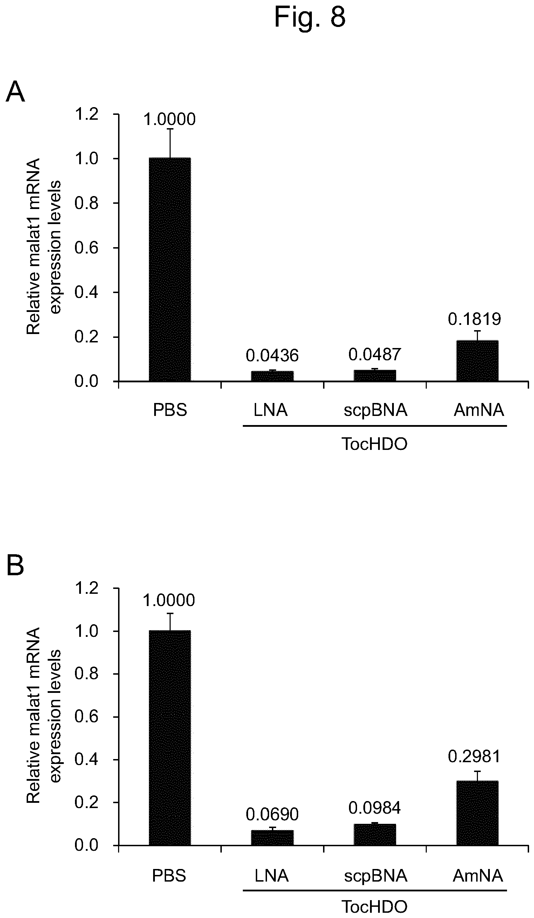

[Figure 8] Figure 8 shows the expression levels of malat1 mRNA in mice intravenously administered in a single dose with double-stranded nucleic acid complex agents. Figure 8A shows the expression levels of mal at 1 mRNA in the musculus quadriceps femoris. Figure 8B shows the expression levels of malat1 mRNA in the cardiac muscle. The error bars show standard errors.

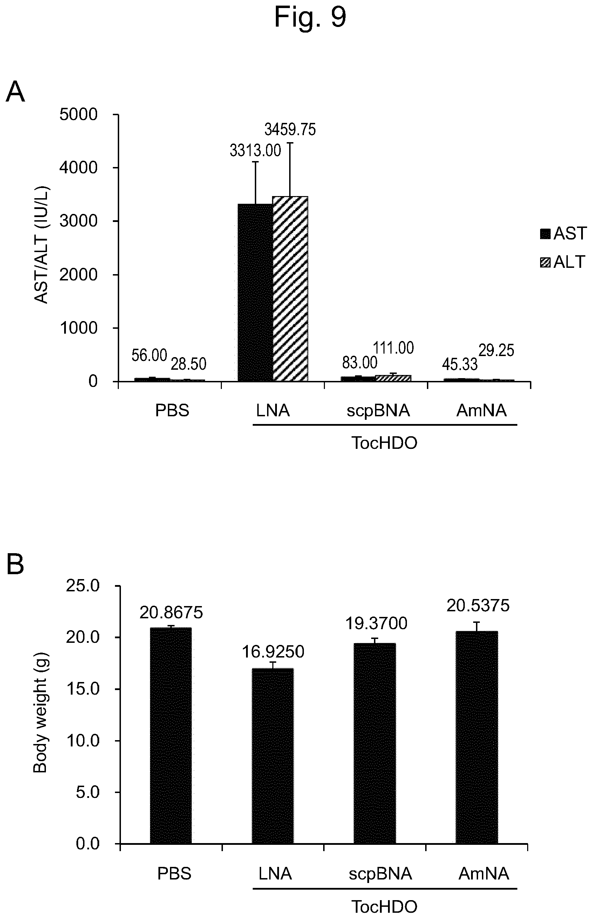

[Figure 9] Figure 9 shows the results of serum analysis and weight measurement of mice intravenously administered in a single dose with double-stranded nucleic acid complex agents targeting malat1. Figure 9A shows the measurement results of AST and ALT in the serum. Figure 9B shows the measurement results of weight. The error bars show standard errors.

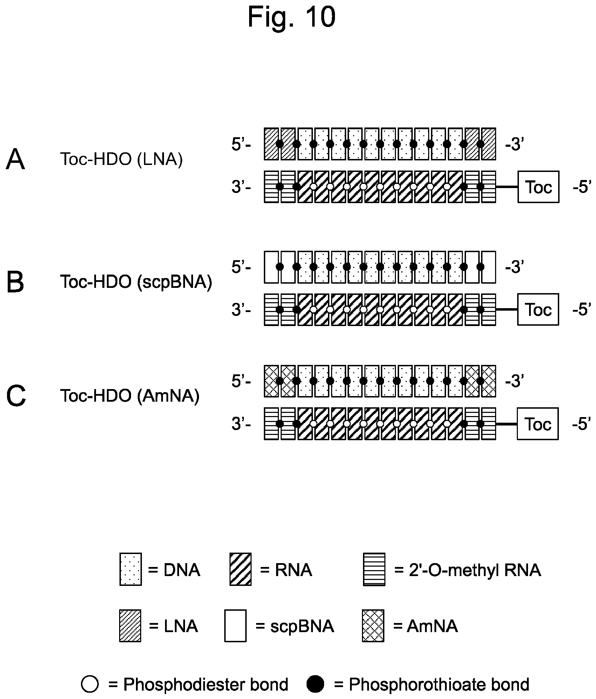

[Figure 10] Figure 10 shows schematic diagrams of structures of nucleic acids used in Examples 3 and 4. Figure 10A shows the structure of Toc-HDO (LNA). Figure 10B shows the structure of Toc-HDO (scpBNA). Figure 10C shows the structure of Toc-HDO (AmNA). Toc represents tocopherol.

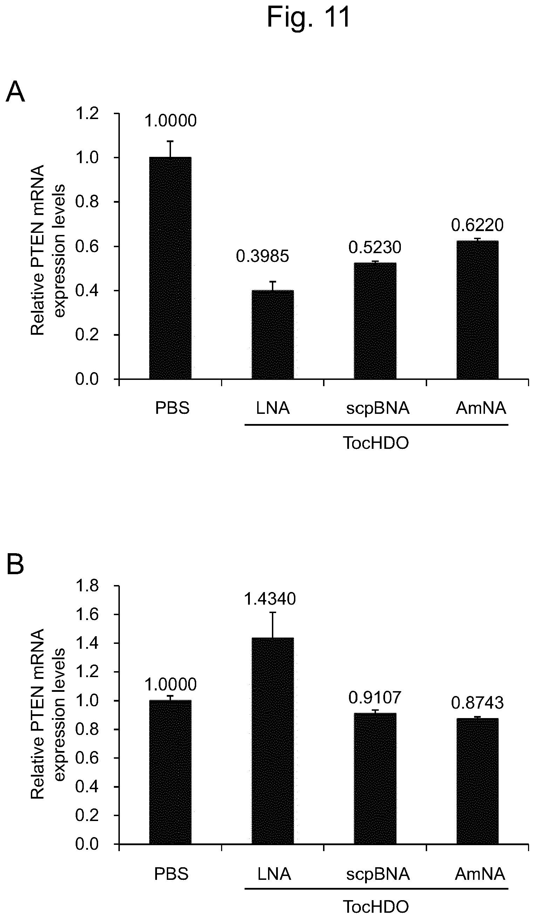

[Figure 11] Figure 11 shows the expression levels of PTEN mRNA in mice intravenously administered in a single dose with double-stranded nucleic acid complex agents. Figure 11A shows the expression level of PTEN mRNA in the liver. Figure 11B shows the expression level of PTEN mRNA in the kidney. The error bars show standard errors.

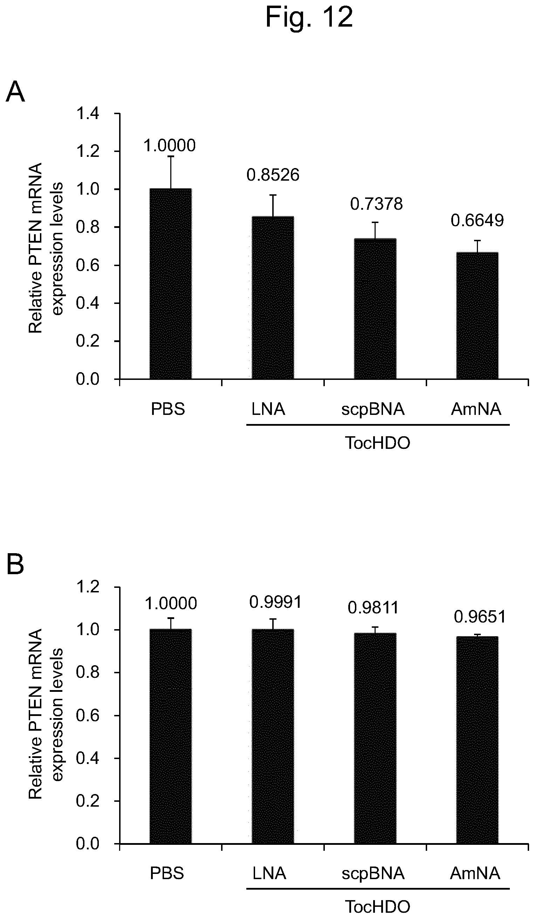

[Figure 12] Figure 12 shows the expression levels of PTEN mRNA in mice intravenously administered in a single dose with double-stranded nucleic acid complex agents. Figure 12A shows the expression level of PTEN mRNA in the musculus quadriceps femoris. Figure 12B shows the expression level of PTEN mRNA in the cardiac muscle. The error bars show standard errors.

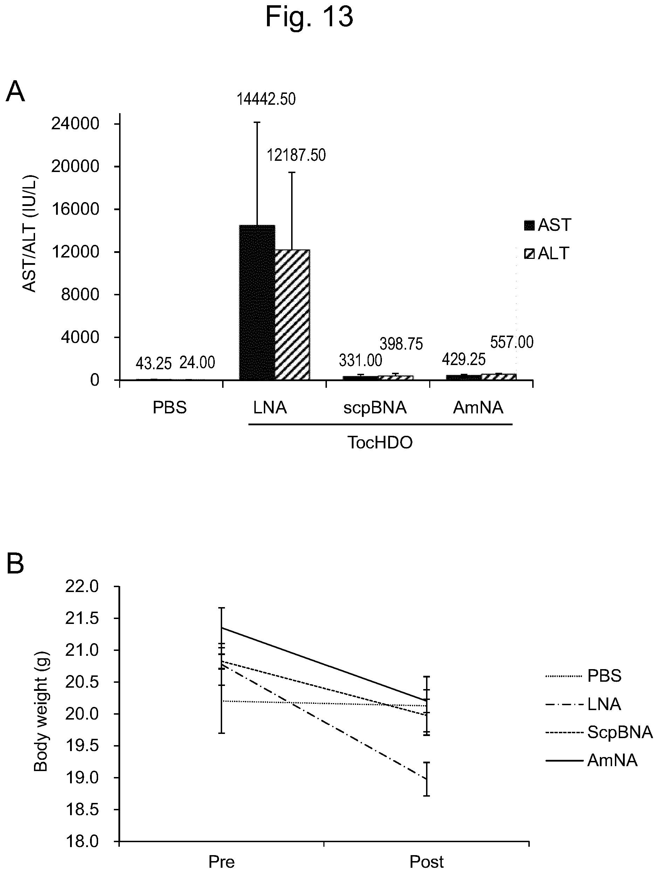

[Figure 13] Figure 13 shows the results of serum analysis and weight measurement of mice intravenously administered in a single dose with double-stranded nucleic acid complex agents targeting PTEN. Figure 13A shows the measurement results of AST and ALT in the serum. Figure 13B shows the weight change before and after administration of a double-stranded nucleic acid complex agent. The error bars show standard errors.

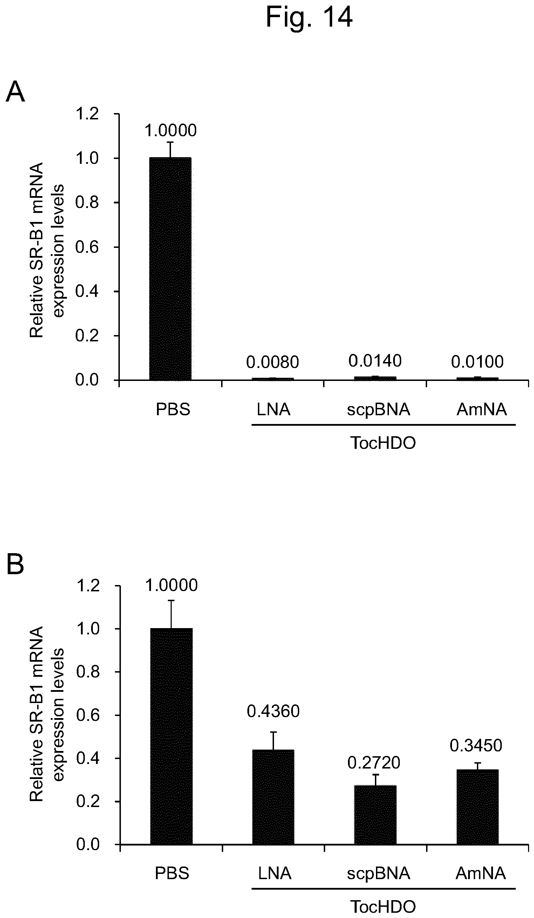

[Figure 14] Figure 14 shows the expression levels of SR-B1 mRNA in mice intravenously administered in a single dose with double-stranded nucleic acid complex agents. Figure 14A shows the expression level of SR-B1 mRNA in the liver. Figure 14B shows the expression level of SR-B 1 mRNA in the kidney. The error bars show standard errors.

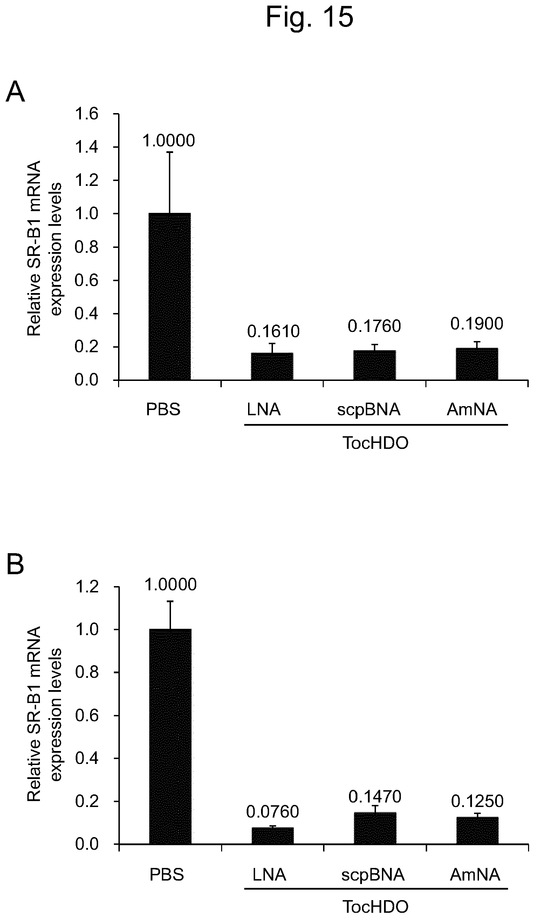

[Figure 15] Figure 15 shows the expression levels of SR-B1 mRNA in mice intravenously administered in a single dose with double-stranded nucleic acid complex agents. Figure 15A shows the expression level of SR-B1 mRNA in the musculus quadriceps femoris. Figure 15B shows the expression level of SR-B1 mRNA in the cardiac muscle. The error bars show standard errors.

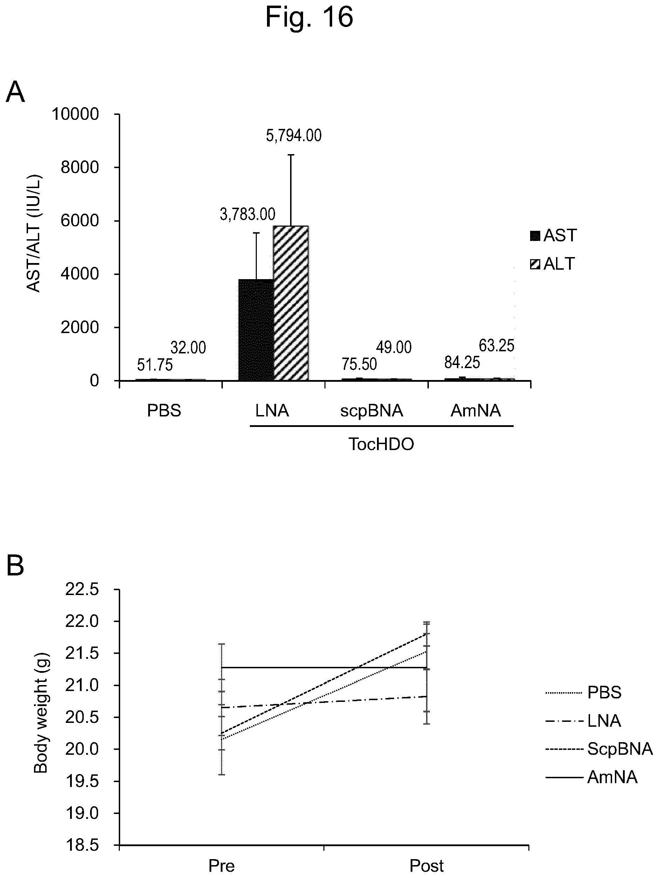

[Figure 16] Figure 16 shows the results of serum analysis and weight measurement of mice intravenously administered in a single dose with double-stranded nucleic acid complex agents targeting SR-B1. Figure 16A shows the measurement results of AST and ALT in the serum. Figure 16B shows the weight change before and after administration of a double-stranded nucleic acid complex agent. The error bars show standard errors.

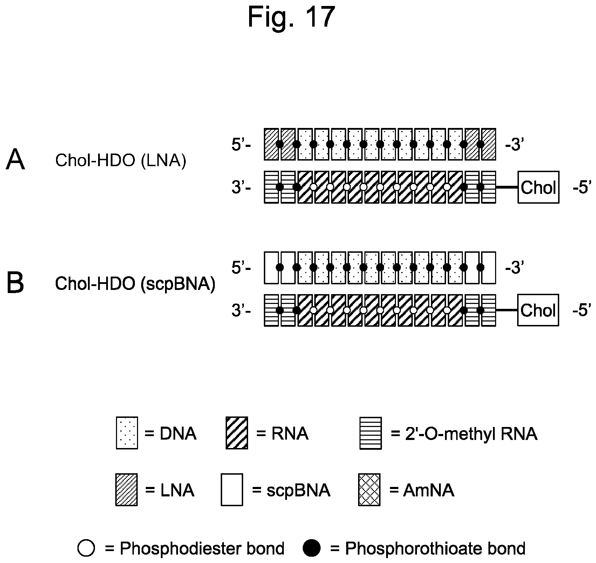

[Figure 17] Figure 17 shows schematic diagrams of nucleic acids used in Example 5. Figure 17A shows the structure of Chol-HDO (LNA). Figure 17B shows the structure of Chol-HDO (scpBNA). Chol represents cholesterol.

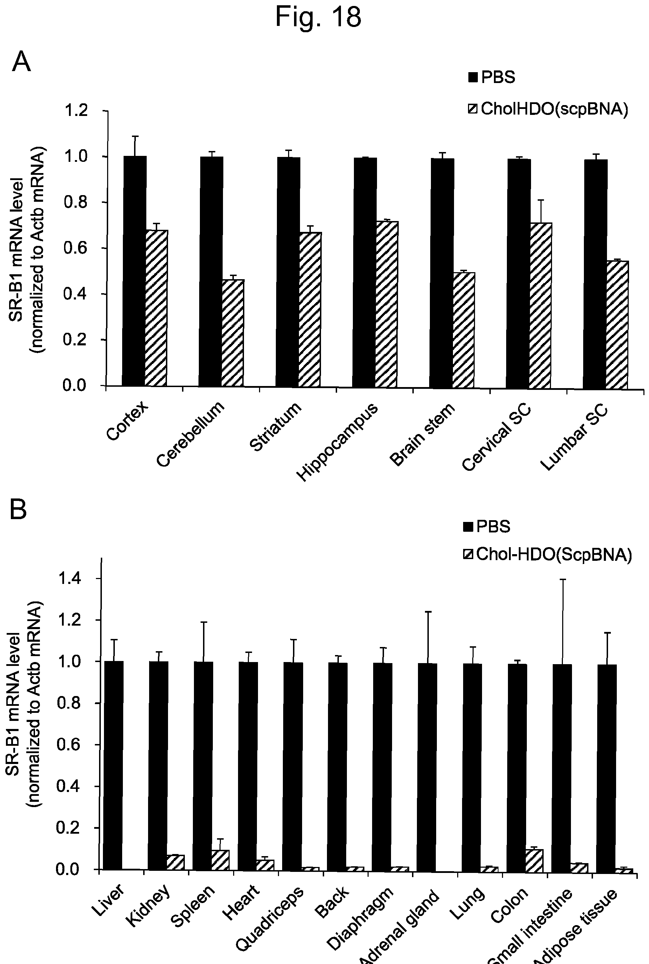

[Figure 18] Figure 18 shows the expression levels of SR-B1 mRNA in mice intravenously administered multiple times with double-stranded nucleic acid complex agents. Figure 18A shows the expression levels of SR-B 1 mRNA in the cortex, cerebellum, striatum, hippocampus, brain stem, cervical spinal cord and lumbar spinal cord. Figure 18B shows the expression levels of SR-B 1 mRNA in the liver, kidney, spleen, heart, quadriceps, back, diaphragm, adrenal gland, lung, colon, small intestine, and adipose tissue. The error bars show standard errors.

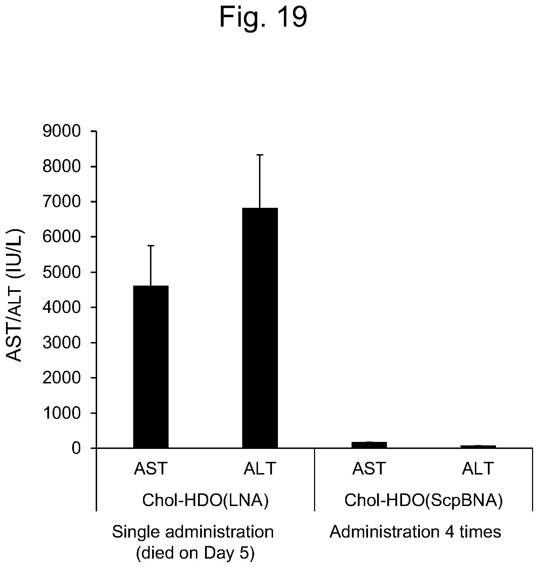

[Figure 19] Figure 19 shows the measurement results of AST and ALT in mice intravenously administered multiple times with double-stranded nucleic acid complex agents. With respect to Chol-HDO (LNA), AST/ALT measurement results of mice administered once are shown since mice died after the first administration.

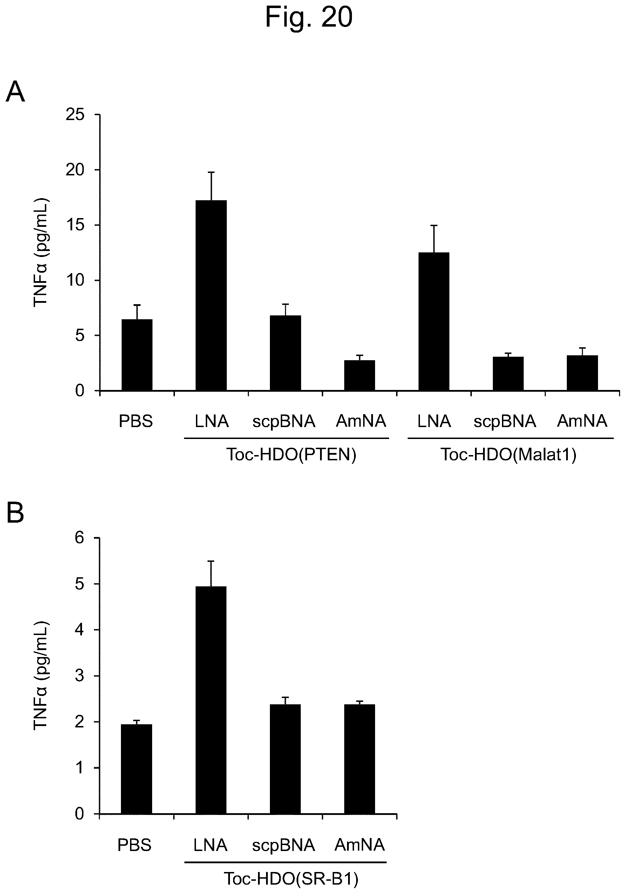

[Figure 20] Figure 20 shows the measurement results of TNFα in the blood of mice intravenously administered in a single dose with double-stranded nucleic acid complex agents.
Figure 20A shows the results of Toc-HDO (PTEN) and Toc-HDO (Malat1). Figure 20B shows the results of Toc-HDO (SR-B1).

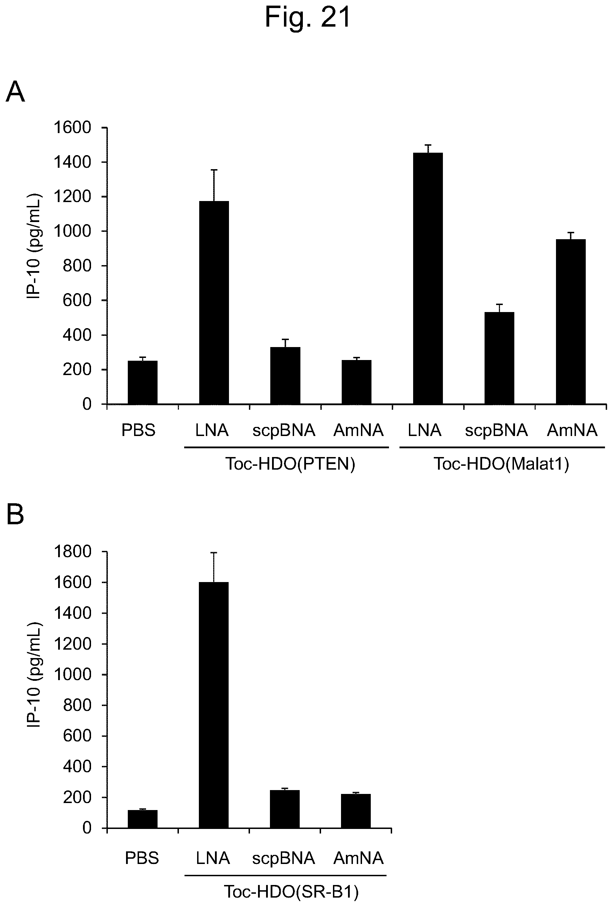

[Figure 21] Figure 21 shows the measurement results of IP-10 in the blood of mice intravenously administered in a single dose with double-stranded nucleic acid complex agents. Figure 21A shows the results of Toc-HDO (PTEN) and Toc-HDO (Malat1). Figure 21B shows the results of Toc-HDO (SR-B1).

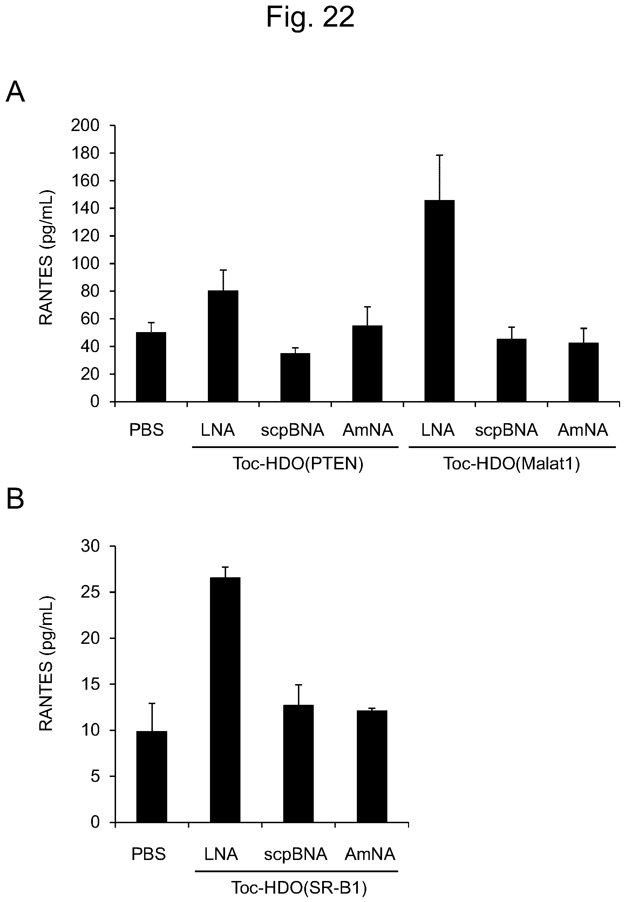

[Figure 22] Figure 22 shows the measurement results of RANTES in the blood of mice intravenously administered in a single dose with double-stranded nucleic acid complex agents.
Figure 22A shows the results of Toc-HDO (PTEN) and Toc-HDO (Malat1). Figure 22B shows the results of Toc-HDO (SR-B1).

### Description of Embodiments

### 1. Double-stranded nucleic acid complex

### 1-1. Overview

A first embodiment of the present invention is a double-stranded nucleic acid complex. The double-stranded nucleic acid complex of the present invention comprises a first nucleic acid strand and a second nucleic acid strand, and the first nucleic acid strand and/or the second nucleic acid strand comprises at least one scpBNA or AmNA. In the double-stranded nucleic acid complex of the present invention, toxicity such as liver toxicity is reduced, and induction of inflammation or gliosis, or an abnormal increase of cytokine or chemokine is reduced.

### 1-2. Definitions of terms

The "transcription product" of a target gene herein refers to any RNA, which is a direct target by the nucleic acid complex of the present invention and synthesized by RNA polymerase. Examples of RNA may include mRNA transcribed from a target gene (*e*.*g*., mature mRNA, mRNA precursor, mRNA without base modification), non-coding RNA (ncRNA) such as miRNA, long non-coding RNA (IncRNA), and natural antisense RNA.

Examples of the "target gene" whose expression is regulated (for example, reduced, changed, or modified) by an antisense effect include, but are not particularly limited to, a gene whose expression level increases in various diseases. Examples of the transcription product of a target gene include SR-B1 mRNA, which is a transcription product of SR-B1 gene, Malat1 non-coding RNA, which is a transcription product of Malat1 gene, and DMPK mRNA, which is a transcription product of DMPK gene. A "target transcription product" may be, for example, the gene of scavenger receptor B 1 (herein often referred to as "SR-B 1 "), myotonic dystrophy protein kinase (herein often referred to as "DMPK"), transthyretin (herein often referred to as "TTR"), apolipoprotein B (herein often referred to as "ApoB"), or metastasis-associated lung adenocarcinoma transcription product 1 (herein often referred to as "Malat1"), for example, non-coding RNA or mRNA thereof. SEQ ID NO: 19 represents the base sequence of mouse Malat1 non-coding RNA, and SEQ ID NO: 20 represents the base sequence of human Malat1 non-coding RNA. SEQ ID NO: 21 represents the base sequence of mouse SR-B1 mRNA, and SEQ ID NO: 22 represents the base sequence of human SR-B1 mRNA. SEQ ID NO: 23 represents the base sequence of mouse DMPK mRNA, and SEQ ID NO: 24 represents the base sequence of human DMPK mRNA. It is noted that, SEQ ID NOs: 19 to 24 each shows a DNA base sequence in place of an mRNA base sequence. The base sequence information of these genes and transcription products are available from databases commonly known, for example, NCBI (the National Center for Biotechnology Information) database (*e*.*g*., GenBank, Trace Archive, Sequence Read Archive, BioSample, BioProject).

An "antisense oligonucleotide (ASO)" or "antisense nucleic acid" herein refers to a single-stranded oligonucleotide containing a base sequence (*i*.*e*, complementary base sequence) capable of hybridizing to at least part of a target transcription product (mainly, a transcription product of a target gene) and producing an antisense effect on the target transcription product. In the double-stranded nucleic acid complex of the present invention, a first nucleic acid strand serves as an ASO, and the target region thereof may contain a 3'UTR, a 5'UTR, an exon, an intron, a coding region, a translation initiation region, a translation termination region, or any other nucleic acid region. The target region of the target transcription product may be at least an 8 base in length, for example, a 10 to 35 base in length, a 12 to 25 base in length, a 13 to 20 base in length, a 14 to 19 base in length or a 15 to 18 base in length, or a 13 to 22 base in length, a 16 to 22 base in length or a 16 to 20 base in length.

The "antisense effect" refers to the effect of controlling the expression or edition of a target transcription product produced by hybridization of ASO to a target transcription product (for example, RNA sense strand). The "controlling the expression or edition of a target transcription product" refers to suppressing or reducing the expression of a target gene or the expression level of a target transcription product ("the expression level of a target transcription product" herein is often referred to as "the level of a target transcription product"), inhibition of translation, RNA edition, splicing function modification effect (for example, splicing switch, exon inclusion, exon skipping), or degradation of a transcription product. For example, in the post-transcription inhibition of a target gene, when an RNA oligonucleotide is introduced as an ASO into a cell, the ASO is annealed with mRNA, *i.e.*, a transcription product of a target gene, to form a partial double strand. The partial double strand plays a role as a cover for preventing translation by a ribosome, with the result that expression of a target protein encoded by a target gene is inhibited at a translational level (steric blocking). On the other hand, when an oligonucleotide containing DNA is introduced as an ASO into a cell, a partial DNA-RNA heteroduplex is formed. The heteroduplex structure is recognized by RNase H, resulting in the degradation of mRNA of a target gene and inhibition of the expression of a protein encoded by the target gene at the level of expression. Furthermore, the antisense effect can be provided on an intron in an mRNA precursor as a target. Moreover, the antisense effect can be provided on miRNA as a target. In this case, since the function of miRNA is inhibited, the expression of a gene normally regulated by the miRNA can be increased. In an embodiment, regulation of the expression of a target transcription product may be a reduction in the level of a target transcription product.

The antisense effect can be measured, for example, by administering a test nucleic acid compound to a subject (for example, mouse) and measuring the expression level of a target gene regulated by the antisense effect provided by the test nucleic acid compound or the level (amount) of a target transcription product (for example, mRNA amount or RNA amount of, *e.g.*, micro RNA, cDNA amount, protein amount), for example, several days later (for example, 2 to 7 days later).

If the measured expression level of a target gene or the level of a target transcription product is decreased by, for example, at least 10%, at least 20%, at least 25%, at least 30%, or at least 40%, compared to the level of a negative control (for example, vehicle administration), it is shown that the test nucleic acid compound can produce an antisense effect (for example, reduction of a target transcription product amount).

The number, types, and positions of non-natural nucleotides in a nucleic acid strand may influence, e.g., an antisense effect provided by a nucleic acid complex. Selection of modification may vary depending on the sequence of, *e.g.*, a target gene. A suitable embodiment can be determined by those skilled in the art with reference to the descriptions of literatures related to an antisense method (for example, WO 2007/143315, WO 2008/043753, and WO 2008/049085). Furthermore, when an antisense effect of a modified nucleic acid complex is measured, if the measured value thus obtained is not significantly low compared to that of the nucleic acid complex before the modification (for example, if the measured value obtained after modification is 70% or more, 80% or more or 90% or more of the measured value of the nucleic acid complex before the modification), relevant modification may be evaluated.

A "translation product of a target gene" herein refers to any polypeptide or protein synthesized by translation of said target transcription product which is a direct target by the nucleic acid complex of the present invention or a transcription product of a target gene.

The term "nucleic acid" or "nucleic acid molecule" used herein may refer to a monomer nucleotide or nucleoside, or an oligonucleotide consisting of a plurality of monomers.

A "nucleic acid strand" or simply "strand" herein refers to an oligonucleotide or a polynucleotide. A nucleic acid strand can be produced as a full-length strand, or a partial strand by a chemical synthesis method, for example using an automatic synthesizing apparatus, or through an enzymatic process using a polymerase, a ligase, or a restriction reaction. Examples of the nucleic acid strand may comprise a natural nucleotide and/or a non-natural nucleotide.

A "nucleoside" generally refers to a molecule consisting of a combination of a base and a sugar. Although not limited, the sugar moiety of a nucleoside is generally composed of pentofuranosyl sugar. Examples of the pentofuranosyl sugar include a ribose and a deoxyribose. The base moiety (nucleobase) of a nucleoside is generally a moiety of a heterocyclic base. Examples of the nucleobase include, but are not limited to, adenine, cytosine, guanine, thymine, uracil and other modified nucleobases (modified bases).

A "nucleotide" refers to a molecule in which a phosphate group is covalently bound to the sugar moiety of said nucleoside. In the case of a nucleotide containing a pentofuranosyl sugar, a phosphate group is usually connected to a hydroxyl group at the 2', 3', or 5' position of the sugar.

An "oligonucleotide" refers to a linear oligomer formed by connecting several or several tens of nucleotides through a covalent bond of a hydroxyl group in the sugar moiety and a phosphate group between adjacent nucleotides. A "polynucleotide" refers to a linear polymer formed by covalently linking a larger number of nucleotides than an oligonucleotide, *i.e.*, several tens or more, preferably several hundreds or more of nucleotides. The phosphate group is generally considered to form an internucleoside bond inside the structure of an oligonucleotide or a polynucleotide.

A "natural nucleoside" herein refers to a nucleoside present in nature. Examples of the natural nucleoside include a ribonucleoside consisting of a ribose and a base such as said adenine, cytosine, guanine, or uracil, and a deoxyribonucleoside consisting of a deoxyribose and a base such as said adenine, cytosine, guanine, or thymine. It is noted that a ribonucleoside found in RNA, and a deoxyribonucleoside found in DNA are herein often referred to as "DNA nucleoside" and "RNA nucleoside", respectively.

A "natural nucleotide" herein refers to a nucleotide present in nature which is a molecule in which a phosphate group is covalently bound to the sugar moiety of said natural nucleoside. Examples of the natural nucleotide include a ribonucleotide known as a constituent unit of RNA and formed by binding a phosphate group to a ribonucleoside, and a deoxyribonucleotide known as a constituent unit of DNA and formed by binding a phosphate group to a deoxyribonucleoside.

A "non-natural nucleotide" herein refers to any nucleotide except a natural nucleotide. Examples of the non-natural nucleotide include a modified nucleotide and a nucleotide mimic. The "modified nucleotide" herein refers to a nucleotide having at least any one of a modified sugar moiety, a modified internucleoside bond, and a modified nucleobase. The "nucleotide mimic" herein comprises a structure to be used to substitute a nucleoside and a bond (linkage) at one or more positions in an oligomer compound. Examples of the nucleotide mimic include a peptide nucleic acid, and a morpholino nucleic acid (morpholino connected via-N(H)-C(=O)-O- or another bond such as a non-phosphodiester bond). The peptide nucleic acid (PNA) is a nucleotide mimic having a main chain in which N-(2-aminoethyl)glycine used in place of a sugar, is bound via an amide bond. A nucleic acid strand comprising a non-natural oligonucleotide herein mostly has desired properties, such as enhanced cellular uptake, enhanced affinity for a target nucleic acid, increased stability in the presence of a nuclease, or increased inhibitory activity. Therefore, a non-natural nucleotide is more preferable than a natural nucleotide.

A "non-natural nucleoside" herein refers to any nucleoside except for a natural nucleoside. Examples of the non-natural nucleoside include a modified nucleoside and a nucleoside mimic. The "modified nucleoside" herein means a nucleoside having a modified sugar moiety and/or a modified nucleobase.

A "mimic" herein refers to a functional group that substitutes a sugar, a nucleobase, and/or an internucleoside bond. In general, a mimic is used in place of a sugar or a combination of a sugar-internucleoside bond, and a nucleobase is maintained for hybridization to a target to be selected. The "nucleoside mimic" herein comprises a structure to be used for substituting a sugar at one or more positions of an oligomer compound, or substituting a sugar and a base, or substituting a bond between monomer subunits constituting an oligomer compound or the like. An "oligomer compound" means a polymer composed of linked monomer subunits that can at least hybridize to a region of a nucleic acid molecule. Examples of a nucleoside mimic include a morpholino, cyclohexenyl, cyclohexyl, tetrahydropyranyl, bicyclic and tricyclic sugar mimics, such as a nucleoside mimic having a non-furanose sugar unit.

The term "modified sugar" refers to a sugar having a substitution and/or any change from a natural sugar moiety (*i.e.*, a sugar moiety found in DNA(2'-H) or RNA(2'-OH)). A "sugar modification" refers to a substitution and/or any change from a natural sugar moiety. A nucleic acid strand herein may contain one or more modified nucleosides including a modified sugar, in some cases. A "sugar-modified nucleoside" refers to a nucleoside having a modified sugar moiety. Such a sugar-modified nucleoside can provide a certain beneficial biological property such as enhanced nuclease stability and increased binding affinity, to a nucleic acid strand. In a certain embodiment, a nucleoside comprises a chemically modified ribofuranose ring moiety. Examples of the chemically modified ribofuranose ring include, but are not limited to, addition of a substituent (comprising 5' and 2' substituents), formation of a bicyclic nucleic acid (bridged nucleic acid, BNA) through bridge formation of a non-geminal ring atom, substitution of a ribosyl ring oxygen atom with S, N(R), or C(R1)(R2) (wherein R, R1, and R2 independently represent H, a C₁-C₁₂ alkyl, or a protecting group), and a combination thereof.

Examples of the sugar-modified nucleoside include, but are not limited to, nucleosides comprising a substituent such as 5'-vinyl, 5'-methyl (R or S), 5'-allyl (R or S), 4'-S, 2'-F (2'-fluoro group), 2'-OCH₃ (2'-OMe group or 2'-O-methyl group), and 2'-O-[2-(N-methylcarbamoyl)ethyl] (2'-O-MCE group), and a 2'-O-methoxyethyl (2'-O-MOE or 2-O(CH₂)₂OCH₃). The substituent at the 2' position can be selected from allyl, amino, azide, thio, -O-allyl, -O-C₁-C₁₀ alkyl, -OCF₃,-O(CH₂)₂SCH₃, -O(CH₂)₂-O-N(Rm)(Rn), and O-CH₂-C(=O)-N(Rm)(Rn), wherein Rm and Rn are each independently H or a substituted or unsubstituted C₁-C₁₀ alkyl. A "2'-modified sugar" means a furanosyl sugar modified at the 2' position. A nucleoside containing a 2'-modified sugar is sometimes referred to as "2'-sugar-modified nucleoside".

A "bicyclic nucleoside" refers to a modified nucleoside comprising a bicyclic sugar moiety. A nucleic acid comprising a bicyclic sugar moiety is generally referred to as a bridged nucleic acid (BNA). A nucleoside comprising a bicyclic sugar moiety is sometimes referred to as a "bridged nucleoside", "bridged non-natural nucleoside" or "BNA nucleoside". Some bridged nucleic acids are shown in Figure 1.

A bicyclic sugar may be a sugar in which the carbon atom at the 2' position and the carbon atom at the 4' position are bridged with two or more atoms. Examples of a bicyclic sugar are known to those skilled in the art. A subgroup of nucleic acids comprising a bicyclic sugar (BNA) or BNA nucleosides can be specified to have a carbon atom at the 2' position and a carbon atom at the 4' position which are bridged with 4'-(CH₂)ₚ-O-2', 4'-(CH₂)ₚ-CH₂-2', 4'-(CH₂)ₚ-S-2', 4'-(CH₂)ₚ-OCO-2', 4'-(CH₂)ₙ-N(R₃)-O-(CH₂)ₘ-2' [wherein p, m, and n represent an integer from 1 to 4, an integer from 0 to 2, and an integer from 1 to 3, respectively; and R₃ represents a hydrogen atom, an alkyl group, an alkenyl group, a cycloalkyl group, an aryl group, an aralkyl group, an acyl group, a sulfonyl group, and a unit substituent (*e*.*g*., a fluorescently or chemiluminescently labeled molecule, a functional group with nucleic acid cleavage activity, and an intracellular or intranuclear localization signal peptide)]. Furthermore, with respect to a BNA or BNA nucleoside in a certain embodiment, in the OR₂ substituent on the carbon atom at the 3' position and the OR₁ substituent on the carbon atom at the 5' position, R₁ and R₂ are typically hydrogen atoms, but may be mutually identical or different, or may also be a phosphate group protected with a protecting group for a hydroxyl group for nucleic acid synthesis, an alkyl group, an alkenyl group, a cycloalkyl group, an aryl group, an aralkyl group, an acyl group, a sulfonyl group, a silyl group, a phosphate group, or P(R₄)R₅ [wherein R₄ and R₅, may be mutually identical or different, and each represent a hydroxyl group, a hydroxyl group protected by a protecting group for nucleic acid synthesis, a mercapto group, a mercapto group protected by a protecting group for nucleic acid synthesis, an amino group, an alkoxy group having 1 to 5 carbon atoms, an alkylthio group having 1 to 5 carbon atoms, a cyanoalkoxy group having 1 to 6 carbon atoms, or an amino group substituted with an alkyl group having 1 to 5 carbon atoms]. Non-limiting examples of such BNA include methyleneoxy(4'-CH₂-O-2') BNA (LNA (Locked Nucleic Acid^{®}, also known as 2',4'-BNA), (*e*.*g*., α-L-methyleneoxy(4'-CH₂O-2') BNA or β-*D-*methyleneoxy(4'-CH₂-O-2') BNA), ethyleneoxy(4'-(CH₂)₂-O-2') BNA (also known as ENA), β-*D*-thio(4'-CH₂-S-2') BNA, aminooxy(4'-CH₂-O-N(R₃)-2') BNA, oxyamino(4'-CH₂-N(R₃)-O-2') BNA (also known as 2',4'-BNA^{NC}; R=H is 2',4'-BNA^{NC}[N-H], R=Me is 2',4'-BNA^{NC}[N-Me]), 2',4'-BNA^{coc}, 3'-amino-2',4'-BNA, 5'-methyl BNA, (4'-CH(CH₃)-O-2') BNA (also known as cEt BNA), (4'-CH(CH₂OCH₃)-O-2') BNA (also known as cMOE BNA), amido BNA (amido-bridged nucleic acid) or (4'-C(O)-N(R)-2') BNA (R=H, or Me) (also known as AmNA; R=H in Figure 1 is AmNA[N-H], R=Me is AmNA[N-Me]), guanidine BNA (also known as GuNA (*e*.*g*., R=H is GuNA[N-H], R=Me is GuNA[N-Me]) in Figure 1), amine BNA (also known as 2'-Amino-LNA) (*e*.*g*., 3-(Bis(3-aminopropyl)amino)propanoyl substituted form), 2'-O,4'-C-spirocyclopropylene-bridged nucleic acid (also known as scpBNA), and other BNAs known to those skilled in the art. Non-limiting examples of such BNA nucleoside include methyleneoxy(4'-CH₂-O-2') BNA nucleoside (LNA nucleoside, also known as 2',4'-BNA nucleoside), (*e*.*g*., α-L-methyleneoxy(4'-CH₂-O-2') BNA nucleoside, or β-D-methyleneoxy(4'-CH₂-O-2') BNA nucleoside), ethyleneoxy(4'-(CH₂)₂-O-2') BNA nucleoside (also known as ENA nucleoside), β-D-thio(4'-CH₂-S-2') BNA nucleoside, aminooxy(4'-CH₂-O-N(R₃)-2') BNA nucleoside, oxyamino(4'-CH₂-N(R₃)-O-2') BNA nucleoside (also known as 2',4'-BNA^{NC} nucleoside; R=H is 2',4'-BNA^{NC}[N-H] nucleoside, R=Me is 2',4'-BNA^{NC}[N-Me] nucleoside), 2',4'-BNA^{COC} nucleoside, 3'-amino-2',4'-BNA nucleoside, 5'-methyl BNA nucleoside, (4'-CH(CH₃)-O-2') BNA nucleoside (also known as cEt nucleoside), (4'-CH(CH₂OCH₃)-O-2') BNA nucleoside (also known as cMOE nucleoside), amide BNA nucleoside, or (4'-C(O)-N(R)-2') BNA nucleoside (R=H, Me) (also known as AmNA nucleoside; R=H in Figure 1 is AmNA[N-H] nucleoside, R=Me is AmNA[N-Me] nucleoside), guanidine BNA nucleoside (also known as GuNA nucleoside (*e*.*g*. R=H is GuNA[N-H] nucleoside, R=Me is GuNA[N-Me] nucleoside in Figure 1)), amine BNA nucleoside (also known as 2'-Amino-LNA nucleoside) (*e*.*g*., 3-(Bis(3-aminopropyl)amino)propanoyl substituted nucleoside), 2'-O,4'-C-spirocyclopropylene-bridged nucleoside (also known as scpBNA nucleoside), and other BNA nucleosides known to those skilled in the art.

The "cationic nucleoside" herein is a modified nucleoside present in the cationic form as compared to the neutral form (*e*.*g*., neutral form of ribonucleoside) at a certain pH (*e.g.*, human physiological pH (about 7.4) or pH of a delivery site (*e*.*g*., organelles, cells, tissues, organs, organisms)). The cationic nucleoside may comprise one or more cationic modified groups at any position of a nucleoside. In an embodiment, the cationic nucleoside is, *e*.*g*., a 2'-Amino-LNA nucleoside (*e*.*g*., 3-(Bis(3-aminopropyl)amino)propanoyl-substituted nucleoside), an aminoalkyl-modified nucleoside (*e*.*g*., 2'-O-methyl and 4'-CH₂CH₂CH₂NH₂-substituted nucleoside) or a GuNA nucleoside (*e*.*g*. R=H in Figure 3 is GuNA[N-H] nucleoside, R=Me is GuNA[N-Me] nucleoside). A bicyclic nucleoside having a methyleneoxy (4'-CH₂-O-2') bridge is sometimes referred to as an LNA nucleoside.

The "modified internucleoside bond" herein refers to an internucleoside bond having a substitution or any change from a naturally occurring internucleoside bond (*i*.*e*., phosphodiester bond). Examples of the modified internucleoside bond include an internucleoside bond containing a phosphorus atom, and an internucleoside bond not containing a phosphorus atom. Examples of a typical phosphorus-containing internucleoside bond include, but are not limited to, a phosphodiester bond, a phosphorothioate bond, a phosphorodithioate bond, a phosphotriester bond (a methylphosphotriester bond or an ethylphosphotriester bond disclosed in U.S. Patent No. 5,955,599), an alkylphosphonate bond (*e*.*g*., a methylphosphonate bond disclosed in U.S. Patent Nos. 5,264,423 and 5,286,717 or a methoxypropylphosphonate bond disclosed in International Publication No. WO 2015/168172), an alkylthiophosphonate bond, a methylthiophosphonate bond, a boranophosphonate bond, an internucleoside bond comprising a cyclic guanidine moiety (*e*.*g*., a substructure represented by the following formula (III): 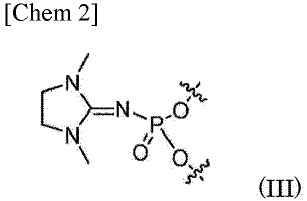 ), an internucleoside bond comprising a guanidine moiety substituted with 1 to 4 C₁₋₆ alkyl groups (*e.g.*, a tetramethylguanidine (TMG) moiety) (*e.g.*, a substructure represented by the following formula (IV): 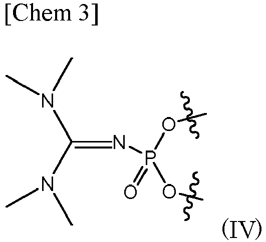 ), an internucleoside bond and a phosphoramidate bond used in a self-neutralizing nucleic acid (ZON) disclosed in International Publication No. WO 2016/081600. A phosphorothioate bond refers to an internucleoside bond in which a non-bridging oxygen atom of a phosphodiester bond is substituted with a sulfur atom. A method for preparing a phosphorus-containing bond or a phosphorus-free bond is well known. A modified internucleoside bond is preferably a bond whose nuclease resistance is higher than that of a naturally occurring internucleoside bond.

In a case where an internucleoside bond has a chiral center, the internucleoside bond may be chirally controlled. The phrase "chirally controlled" means that it is present as a single diastereomer with respect to a chiral center, for example, chiral-bound phosphorus. An internucleoside bond which is chirally controlled may be completely pure in chirality or may have high chiral purity, for example, a chiral purity of 90%de, 95%de, 98%de, 99%de, 99.5%de, 99.8%de, or 99.9%de or more. The term "chiral purity" herein refers to a proportion of one diastereomer in a mixture of diastereomers and is expressed as diastereomeric excess (%de) which is defined as (diastereomers of interest - other diastereomers)/(all diastereomers) × 100 (%).

For example, an internucleoside bond may be a phosphorothioate bond which is chirally controlled into Rp or Sp configuration, an internucleoside bond (*e*.*g*., a partial structure represented by formula (IV)) comprising a guanidine moiety substituted with 1 to 4 C₁₋₆ alkyl groups (*e*.*g*., tetramethylguanidine (TMG) moiety; see, *e*.*g*., Alexander A. Lomzov et al., Biochem Biophys Res Commun., 2019, 513(4), 807-811), and/or an internucleoside bond (*e*.*g*., a partial structure represented by formula (III)) comprising a cyclic guanidine moiety. A method for preparing an internucleoside bond which is chirally controlled is known. For example, a phosphorothioate bond which is chirally controlled into Rp or Sp configuration can be synthesized according to the methods described in Naoki Iwamoto et al., Angew. Chem. Int. Ed. Engl. 2009, 48(3), 496-9, Natsuhisa Oka et al., J. Am. Chem. Soc. 2003, 125, 8307-8317, Natsuhisa Oka et al., J. Am. Chem. Soc. 2008, 130, 16031-16037, Yohei Nukaga et al., J. Org. Chem. 2016, 81, 2753-2762, Yohei Nukaga et al., J. Org. Chem. 2012, 77, 7913-7922. A phosphorothioate bond which is chirally controlled into Rp or Sp configuration is also known, and known to have effects as disclosed in Naoki Iwamoto et al., Nat. Biotechnol, 2017, 35(9), 845-851 and Anastasia Khvorova et al., Nat. Biotechnol., 2017, 35(3), 238-248. For example, in an embodiment, a phosphorothioate bond which is chirally controlled into Sp configuration is more stable than that those in Rp configuration, and/or ASO which is chirally controlled into an Sp configuration promotes cleavage of target RNA with RNase H1 and brings a more sustained response *in vivo.* A method for preparing an internucleoside bond comprising a guanidine moiety substituted with 1 to 4 C₁₋₆ alkyl groups (*e*.*g*., TMG moiety) is known, and can be synthesized according to, for example, the method disclosed in Alexander A. Lomzov et al., Biochem Biophys Res Commun., 2019, 513(4), 807-811.

The term "nucleobase" or "base" used herein refers to a base component (heterocyclic part) constituting a nucleic acid and adenine, guanine, cytosine, thymine, and uracil are mainly known. The term "nucleobase" or "base" herein includes both of modified and unmodified nucleobases (bases) unless otherwise specified. Therefore, a purine base may be either a modified or unmodified purine base, unless otherwise specified. A pyrimidine base may be either a modified or unmodified pyrimidine base, unless otherwise specified.

A "modified nucleobase" or a "modified base" means any nucleobase other than adenine, cytosine, guanine, thymine, or uracil. An "unmodified nucleobase" or an "unmodified base" (natural nucleobase) means adenine (A) and guanine (G), which are purine bases, as well as thymine (T), cytosine (C), and uracil (U), which are pyrimidine bases. Examples of the modified nucleobase include, but are not limited to, 5-methylcytosine, 5-fluorocytosine, 5-bromocytosine, 5-iodocytosine or N4-methylcytosine; N6-methyladenine or 8-bromoadenine; 2-thio-thymine; and N2-methylguanine or 8-bromoguanine. A modified nucleobase is preferably 5-methylcytosine.

The term "complementary" used herein refers to a relationship in which nucleobases can form a so-called Watson-Crick base pair (natural base pair) or a non-Watson-Crick base pair (Hoogsteen base pair and the like.) via a hydrogen bond. The antisense oligonucleotide region in the first nucleic acid strand is not necessarily required to be completely complementary to at least part of a target transcription product (*e*.*g*., a transcription product of a target gene), and it is acceptable if the base sequence has complementarity of at least 70%, preferably at least 80%, and further preferably at least 90% (*e.g.*, 95%, 96%, 97%, 98%, or 99% or more). An antisense oligonucleotide region in the first nucleic acid strand can hybridize to a target transcription product, when the base sequence is complementary (typically, when the base sequence is complementary to at least part of the base sequence of the target transcription product). Similarly, it is not necessarily required that a complementary region in the second nucleic acid strand is completely complementary to at least part of the first nucleic acid strand, but it is acceptable if the base sequence has a complementarity of at least 70%, preferably at least 80%, and more preferably at least 90% (*e.g.*, 95%, 96%, 97%, 98%, or 99% or more). When the base sequence of the complementary region in the second nucleic acid strand is complementary to at least part of the first nucleic acid strand, the region can be annealed. The complementarity of a base sequence can be determined by *e*.*g*., BLAST program. Those skilled in the art can readily determine the conditions (*e*.*g*., temperature, salt concentration) under which two strands can be annealed or hybridized to each other, taking the complementarity between the strands into account. Further, those skilled in the art can readily design an antisense nucleic acid which is complementary to a target transcription product based on, for example, information on the base sequence of a target gene.

Hybridization conditions may be a variety of stringent conditions, such as a low-stringent condition and a high-stringent condition. A low-stringent condition may be a condition with a relatively low temperature and a high salt concentration, such as 30°C, 2 × SSC, and 0.1% SDS. A high-stringent condition may be a condition with a relatively high temperature and a low salt concentration, such as 65°C, 0.1 × SSC, and 0.1% SDS. The stringency of hybridization can be adjusted by varying the conditions, such as temperature and salt concentration. Here, 1 × SSC contains 150 mM of sodium chloride and 15 mM of sodium citrate.

A "subject" herein refers to the object to which the double-stranded nucleic acid complex or pharmaceutical composition of the present invention is applied. A subject comprises an individual as well as an organ, a tissue, and a cell. When the subject is an individual, any animal including a human may be applicable. For example, in addition to a human, a variety of domestic animals, domestic fowls, pets, and laboratory animals are included. Although not limited, the subject may be an individual who requires reduction of the expression level of a target transcription product or an individual who requires treatment or prevention of a disease.

### 1-3. Configuration

The double-stranded nucleic acid complex of the present invention comprises a first nucleic acid strand and a second nucleic acid strand. The specific configurations of individual nucleic acid strands are shown below.

In the double-stranded nucleic acid complex of the present invention, the first nucleic acid strand is capable of hybridizing to at least part of a target gene or a transcription product thereof, and has an antisense effect on the target gene or transcription product thereof, the second nucleic acid strand comprises a base sequence complementary to the first nucleic acid strand, and the first nucleic acid strand and/or said second nucleic acid strand comprises at least one bridged non-natural nucleoside represented by the following formula (I) or formula (II): 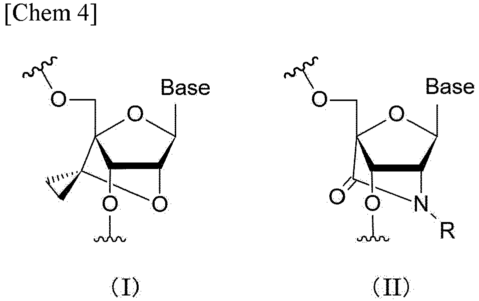 wherein R represents a hydrogen atom or a methyl group.

A bridged non-natural nucleoside represented by the above formula (I) is 2'-O,4'-C-spirocyclopropylene-bridged nucleic acid and is expressed mainly as "scpBNA" herein.

A bridged non-natural nucleoside represented by the above formula (II) is amide BNA (amido-bridged nucleic acid) and can be expressed as (4'-C (O)-N(R)-2')BNA (R=H, Me) but herein mainly expressed as "AmNA". In the above formula (II), R may be either a hydrogen atom or a methyl group. Unless otherwise specified, R herein may be either a hydrogen atom or a methyl group, but when the two are distinguished, the bridged non-natural nucleoside can be expressed as AmNA[N-H] when R is a hydrogen atom, and the bridged non-natural nucleoside can be expressed as AmNA[N-Me] when R is a methyl group.

The number of bridged non-natural nucleosides represented by the formula (I) or formula (II) described above which are contained in a first nucleic acid strand and/or a second nucleic acid strand constituting a double-stranded nucleic acid complex is at least one and may be, for example, 2 or more, 3 or more, 4 or more, 5 or more, 6 or more, 7 or more, 8 or more, 9 or more, or 10 or more and 30 or less, 25 or less, 20 or less, 15 or less, 10 or less, 9 or less, 8 or less, 7 or less, 6 or less, 5 or less, 4 or less, 3 or less, or 2 or less. The number of bridged non-natural nucleosides represented by the formula (I) or formula (II) may be in the range of, for example, 1 to 10, and preferably 1 to 6. The number may be, for example, 1, 2, 3, 4, 5, or 6.

A first nucleic acid strand and/or a second nucleic acid strand constituting a double-stranded nucleic acid complex of the present invention may comprise only a bridged non-natural nucleoside represented by formula (I) or a bridged non-natural nucleoside represented by formula (II), or both of them, of those represented by formulas (I) and (II).

Furthermore, the bridged non-natural nucleosides represented by formula (I) and formula (II) described above may be contained only in the first nucleic acid strand, only in the second nucleic acid strand, or in both the first nucleic acid strand and the second nucleic acid strand.

The base lengths of a first nucleic acid strand and a second nucleic acid strand are not particularly limited and they may be at least 8 base in length, at least 9 base in length, at least 10 base in length, at least 11 base in length, at least 12 base in length, at least 13 base in length, at least 14 base in length, or at least 15 base in length. The base lengths of a first nucleic acid strand and a second nucleic acid strand may be 35 base in length or less, 30 base in length or less, 25 base in length or less, 24 base in length or less, 23 base in length or less, 22 base in length or less, 21 base in length or less, 20 base in length or less, 19 base in length or less, 18 base in length or less, 17 base in length or less, or 16 base in length or less. The base lengths of a first nucleic acid strand and a second nucleic acid strand may be the same or different (for example, either one of them may be shorter or longer by 1 to 3 bases). A double-strand structure formed by a first nucleic acid strand and a second nucleic acid strand may contain a bulge. The length may be selected in consideration of, for example, factors such as cost and synthesis yield, and in particular, the balance between the strength of an antisense effect and the specificity of a nucleic acid strand to a target.

A first nucleic acid strand, when it is hybridized to a target transcription product, can comprise at least 4, at least 5, at least 6, or at least 7 consecutive nucleosides recognized by RNase H. Usually, the region may be a region comprising consecutive nucleosides of 4 to 20 bases, 5 to 16 bases, or 6 to 12 bases. As the nucleosides recognized by RNase H, for example, natural deoxyribonucleosides can be used. Modified deoxyribonucleosides and suitable nucleosides containing other bases are well known in the field. It is also known that nucleosides such as ribonucleoside having a hydroxy group at the 2' position are inappropriate as said nucleosides. With respect to applicability to this region comprising "at least 4 consecutive nucleosides", the suitability of the nucleosides can be readily determined. In an embodiment, a first nucleic acid strand may comprise at least 4 consecutive deoxyribonucleosides.

In an embodiment, the nucleosides in a first nucleic acid strand comprise deoxyribonucleosides or consist thereof. For example, 70% or more, 80% or more, 90% or more, or 95% or more of the nucleosides in a first nucleic acid strand are deoxyribonucleosides.

In an embodiment, the first nucleic acid strand may be a gapmer. The "gapmer" herein refers to, in principle, a single-stranded nucleic acid consisting of a central region (DNA gap region) and wing regions directly positioned at the 5' end and the 3' end (referred to as 5'-wing region and 3'-wing region, respectively). The length of the DNA gap region may be 13 to 22 base in length, 16 to 22 base in length or 16 to 20 base in length, or 4 to 20 base in length, 5 to 18 base in length, 6 to 16 base in length, 7 to 14 base in length or 8 to 12 base in length. Said central region of a gapmer comprises at least 4 consecutive deoxyribonucleosides and said wing regions comprise at least one non-natural nucleoside. Although not limited, the non-natural nucleoside contained in the wing region usually has a higher binding strength to RNA than a natural nucleoside and is highly resistant to a nucleolytic enzyme (*e*.*g*., nuclease). If the non-natural nucleoside constituting a wing region comprises or consists of a bridged nucleoside, said gapmer is referred to particularly as "BNA/DNA gapmer". The number of a bridged nucleoside contained in the 5'-wing region and 3'-wing region is at least 1 and may be, for example, 2 or 3. The bridged nucleoside contained in the 5'-wing region and 3'-wing region may be present continuously or non-continuously in the 5'-wing region and the 3'-wing region. The bridged nucleoside may further comprise a modified nucleobase (for example, 5-methylcytosine). If the bridged nucleoside is LNA nucleoside, said gapmer is referred to as "LNA/DNA gapmer". If the non-natural nucleoside constituting the 5'-wing region and 3'-wing region comprises a peptide nucleic acid or consists of a peptide nucleic acid, said gapmer is particularly referred to as a "peptide nucleic acid gapmer". If the non-natural nucleoside constituting the 5'-wing region and 3'-wing region comprises a morpholino nucleic acid or consists of a morpholino nucleic acid, said gapmer is particularly referred to as a "morpholino nucleic acid gapmer". The base lengths of the 5'-wing region and 3'-wing region may be each independently at least 2 base in length, for example, 2 to 10 base in length, 2 to 7 base in length, or 3 to 5 base in length. In an embodiment, the 5'-wing region and/or 3'-wing region may comprise at least one type of non-natural nucleoside and may further comprise a natural nucleoside. The 5'-wing region and 3'-wing region may be non-natural nucleosides, such as 2'-O-methyl modified nucleosides, connected via a modified internucleoside bond such as a phosphorothioate bond.

A first nucleic acid strand constituting said gapmer may be composed of bridged nucleosides of 2 to 7 base in length or 3 to 5 base in length (for example, 2 or 3 base in length), ribonucleosides or deoxyribonucleosides of 4 to 15 base in length or 8 to 12 base in length (for example, 8 or 10 base in length), and bridged nucleosides of 2 to 7 base in length or 3 to 5 base in length (for example, 2 or 3 base in length), in this order from the 5' end.

It is noted that a nucleic acid strand having a wing region at only one side of the 5'-end side or 3'-end side is called a "hemi gapmer" in the technical field, but a hemi gapmer is encompassed in a gapmer herein.

The 5'-wing region and/or 3'-wing region of a first nucleic acid strand may comprise a bridged non-natural nucleoside represented by formula (I) or formula (II) described above. The number of bridged non-natural nucleosides represented by formula (I) or formula (II) described above contained in each of the 5'-wing region or 3'-wing region may be at least 1, for example, 2 or more, or 3 or more and may be 5 or less, 4 or less, 3 or less, or may be 2 or less. The number may be, for example, 1, 2, 3, or 4. In an embodiment, the 5'-wing region and/or 3'-wing region of a first nucleic acid strand may consist of bridged non-natural nucleosides represented by formula (I) or formula (II) described above.

In an embodiment, the 5'-wing region and/or 3'-wing region of a first nucleic acid strand comprises a bridged non-natural nucleoside represented by the formula (I) or formula (II) described above and an LNA nucleoside.

In the double-stranded nucleic acid complex of the present invention, the first nucleic acid strand may be a mixmer. The "mixmer" herein refers to a nucleic acid strand comprising natural nucleosides and non-natural nucleosides having a random segment length periodically or alternately and not comprising 4 or more consecutive deoxyribonucleosides and ribonucleosides. A mixmer in which said non-natural nucleosides are bridged nucleosides and the natural nucleosides are deoxyribonucleosides is particularly referred to as "BNA/DNA mixmer". Said bridged nucleoside may be a bridged non-natural nucleoside represented by the formula (I) or formula (II) described above. In the mixmer, a mixmer in which said non-natural nucleoside is a peptide nucleic acid, and the natural nucleoside is a deoxyribonucleoside is particularly referred to as a "peptide nucleic acid/DNA mixmer". In the mixmer, a mixmer in which said non-natural nucleoside is a morpholino nucleic acid and the natural nucleoside is a deoxyribonucleoside is particularly referred to as a "morpholino nucleic acid/DNA mixmer". A mixmer is not limited to contain only two types of nucleosides. A mixmer may contain any number of types of nucleosides regardless of whether the nucleoside is a natural, modified nucleoside or a nucleoside mimic. For example, a mixmer may have a single deoxyribonucleoside or 2 consecutive deoxyribonucleosides separated by a bridged nucleoside (for example, an LNA nucleoside, or a bridged non-natural nucleoside represented by the formula (I) or formula (II) described above). A bridged nucleoside may further contain a modified nucleobase (for example, 5-methylcytosine).

All nucleosides in a second nucleic acid strand may be composed of ribonucleosides and/or modified nucleosides. For example, all nucleosides in a second nucleic acid strand may be composed of ribonucleosides. All nucleosides in a second nucleic acid strand may be composed of deoxyribonucleosides and/or modified nucleosides, and may not contain a ribonucleoside.

In an embodiment, a second nucleic acid strand may comprise at least 4 consecutive ribonucleosides complementary to the above-described at least 4 consecutive nucleosides (for example, deoxyribonucleosides) in the central region of a first nucleic acid strand. This is because the second nucleic acid strand and the first nucleic acid strand form a partial DNA-RNA heteroduplex that is recognized by RNaseH for undergoing cleavage. The at least 4 consecutive ribonucleosides in a second nucleic acid strand are preferably linked with a naturally occurring internucleoside bond, *i.e.*, a phosphodiester bond.

In another embodiment, a second nucleic acid strand may further comprise at least 2 consecutive deoxyribonucleosides in addition to the at least 4 consecutive ribonucleosides described above. The at least 2 consecutive deoxyribonucleosides are complementary to a first nucleic acid strand and may be contained in the region complementary to the central region of a first nucleic acid strand. The at least 2 consecutive deoxyribonucleosides may be positioned at either one of the 5' side and 3' side or at both the 5' side and 3' side of the at least 4 consecutive ribonucleosides described above. The at least 2 consecutive deoxyribonucleosides may be 2, 3, 4, 5, or 6 or more consecutive deoxyribonucleosides.

At least 1, at least 2 (for example, 2), at least 3, or at least 4 nucleosides from an end (the 5' end, 3' end, or both ends) of a second nucleic acid strand may be modified nucleosides. The modified nucleosides may comprise a modified sugar and/or a modified nucleobase. The modified sugar may be 2'-modified sugar (for example, sugar containing a 2'-O-methyl group). The modified nucleobase may also be 5-methylcytosine.

A second nucleic acid strand may be composed of modified nucleosides (for example, modified nucleosides containing a 2'-modified sugar) of 2 to 7 base in length or 3 to 5 base in length (for example, 2 or 3 base in length), ribonucleosides or deoxyribonucleosides (optionally linked via a modified internucleoside bond) of 4 to 15 base in length or 8 to 12 base in length (for example, 8 or 10 base in length), and modified nucleosides (for example, modified nucleosides containing a 2' modified sugar) of 2 to 7 base in length or 3 to 5 base in length (for example, 2 or 3 base in length), in this order from the 5' end. In this case, the first nucleic acid strand may be a gapmer.

In an embodiment, a second nucleic acid strand may comprise a bridged non-natural nucleoside represented by the formula (I) or formula (II) described above in the region consisting of a base sequence complementary to the 5'-wing region and/or 3'-wing region of a first nucleic acid strand.

The number of bridged nucleosides other than bridged non-natural nucleosides represented by the formula (I) or formula (II) described above contained in a first nucleic acid strand and/or a second nucleic acid strand constituting a double-stranded nucleic acid complex is not limited and the number is, for example, 0 to 50, 0 to 40, 0 to 30, 0 to 20, 0 to 15, 0 to 12, 0 to 10, 0 to 8, or 0 to 6, preferably 0 to 5, for example, 0, 1, 2, 3, 4, or 5.

Furthermore, the number of non-natural nucleosides other than bridged non-natural nucleosides represented by the formula (I) or formula (II) described above contained in a first nucleic acid strand and/or a second nucleic acid strand constituting a double-stranded nucleic acid complex is not limited and the number is, for example, 0 to 30, 0 to 20, 0 to 15, 0 to 12, 0 to 10, 0 to 8, or 0 to 6, and preferably, 0 to 5, for example, 0, 1, 2, 3, 4, or 5.

In an embodiment, a first nucleic acid strand and/or a second nucleic acid strand constituting a double-stranded nucleic acid complex may comprise at least one ribose 2' position-modified nucleoside in addition to a bridged non-natural nucleoside represented by the formula (I) or formula (II) described above. The ribose 2' position-modified nucleoside may be at least one ribose 2' position-modified nucleoside selected from the group consisting of a 2'-O-methyl modified nucleoside (2'-O-Me-modified nucleoside), a 2'-O-methoxyethyl modified nucleoside (2'-O-MOE modified nucleoside), and a 2'-O-[2-(N-methylcarbamoyl)ethyl] modified nucleoside (2'-O-MCE-modified nucleoside).

In an embodiment, a first nucleic acid strand and/or a second nucleic acid strand constituting a double-stranded nucleic acid complex comprises at least one bridged non-natural nucleoside represented by the formula (I) or formula (II) described above and at least one 2'-O-methyl modified nucleoside (2'-O-Me-modified nucleoside).

In an embodiment, a first nucleic acid strand and/or a second nucleic acid strand constituting a double-stranded nucleic acid complex comprises at least one bridged non-natural nucleoside represented by the formula (I) or formula (II) described above and at least one 2'-O-methoxyethyl modified nucleoside (2'-O-MOE modified nucleoside).

In an embodiment, a first nucleic acid strand and/or a second nucleic acid strand constituting a double-stranded nucleic acid complex comprises at least one bridged non-natural nucleoside represented by the formula (I) or formula (II) described above and at least one 2'-O-[2-(N-methylcarbamoyl)ethyl] modified nucleoside (2'-O-MCE-modified nucleoside).

In an embodiment, the 5'-wing region of a first nucleic acid strand may comprise at least one bridged non-natural nucleoside represented by the formula (I) or formula (II) described above, and at least one ribose 2' position-modified nucleoside at the 3' side thereof, and the 3'-wing region of the first nucleic acid strand may comprise at least one bridged non-natural nucleoside represented by the formula (I) or formula (II) described above, and at least one ribose 2' position-modified nucleoside at the 5' side thereof.

In an embodiment, the 5'-wing region of a first nucleic acid strand may comprise at least one bridged non-natural nucleoside represented by the formula (I) or formula (II) described above, and at least one ribose 2' position-modified nucleoside at the 5' side thereof, and the 3'-wing region of the first nucleic acid strand may comprise at least one bridged non-natural nucleoside represented by the formula (I) or formula (II) described above, and at least one ribose 2' position-modified nucleoside at the 5' side thereof.

In an embodiment, the 5'-wing region of a first nucleic acid strand may comprise at least one bridged non-natural nucleoside represented by the formula (I) or formula (II) described above, and at least one ribose 2' position-modified nucleoside at the 3' side thereof, and the 3'-wing region of the first nucleic acid strand may comprise at least one bridged non-natural nucleoside represented by the formula (I) or formula (II) described above, and at least one ribose 2' position-modified nucleoside at the 3' side thereof.

In a preferable embodiment, the 5'-wing region of a first nucleic acid strand may comprise at least one bridged non-natural nucleoside represented by the formula (I) or formula (II) described above, and at least one ribose 2' position-modified nucleoside at the 5' side thereof, and the 3'-wing region of the first nucleic acid strand may comprise at least one bridged non-natural nucleoside represented by the formula (I) or formula (II) described above, and at least one ribose 2' position-modified nucleoside at the 3' side thereof.

A first nucleic acid strand and/or a second nucleic acid strand may comprise a nucleoside mimic or a nucleotide mimic entirely or in a part. The nucleotide mimic may be a peptide nucleic acid and/or a morpholino nucleic acid.

The internucleoside bond in a first nucleic acid strand and a second nucleic acid strand may be a naturally occurring internucleoside bond and/or modified internucleoside bond. Although not limited, it is preferable that at least 1, at least 2, at least 3 internucleoside bonds from an end of a first nucleic acid strand and/or second nucleic acid strand (the 5' end, 3' end, or both ends) is a modified internucleoside bond. For example, the two internucleoside bond from an end of a nucleic acid strand refers to an internucleoside bond closest to the end of the nucleic acid strand and an internucleoside bond adjacent thereto and positioned to the opposite side to the end. The modified internucleoside bond in a terminal region of a nucleic acid strand is preferable since undesirable degradation of the nucleic acid strand can be reduced or inhibited.

In an embodiment, the internucleoside bonds in the entirety of or in a part of a first nucleic acid strand and/or a second nucleic acid strand may be a modified internucleoside bond. In an embodiment, a first nucleic acid strand and/or a second nucleic acid strand may each comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more of modified internucleoside bonds. In an embodiment, a first nucleic acid strand and/or a second nucleic acid strand each may comprise a modified internucleoside bond in a ratio of at least 5%, at least 10%, at least 20%, at least 30%, at least 40%, at least 50%, at least 60%, at least 70%, at least 80%, at least 90%, at least 93%, at least 95%, at least 98%, or 100%. In an embodiment, the modified internucleoside bond may be a phosphorothioate bond.

In an embodiment, a first nucleic acid strand and/or a second nucleic acid strand each may comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more of internucleoside bonds which are chirally controlled. In an embodiment, a first nucleic acid strand and/or a second nucleic acid strand each may comprise internucleoside bonds which are chirally controlled in a ratio of at least 5%, at least 10%, at least 20%, at least 30%, at least 40%, at least 50%, at least 60%, at least 70%, at least 80%, at least 90%, or more.

In an embodiment, a first nucleic acid strand and/or a second nucleic acid strand each may comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more of non-negatively charged internucleoside bonds (preferably, neutral internucleoside bond). In an embodiment, a first nucleic acid strand and/or a second nucleic acid strand each may comprise a non-negatively charged internucleoside bond in a ratio of at least 5%, at least 10%, at least 20%, at least 30%, at least 40%, at least 50%, at least 60%, at least 70%, at least 80%, at least 90% or more.

In an embodiment, at least 1, at least 2, or at least 3 internucleoside bonds from the 5' end of a second nucleic acid strand may be modified internucleoside bonds. At least 1, at least 2, or at least 3 internucleoside bonds from the 3' end of a second nucleic acid strand may be modified internucleoside bonds such as a phosphorothioate bond, an internucleoside bond containing a guanidine moiety (for example, TMG moiety) substituted with 1 to 4 C₁₋₆ alkyl groups (*e*.*g*., a partial structure represented by formula (IV)) and/or an internucleoside bond containing a cyclic guanidine moiety (*e*.*g*., a partial structure represented by formula (III)). The modified internucleoside bond may be chirally controlled in Rp or Sp configuration.

At least 1 (for example, 3) internucleoside bond from the 3' end of a second nucleic acid strand may be a modified internucleoside bond highly resistant to RNase, such as a phosphorothioate bond. A modified internucleoside bond such as a phosphorothioate bond is preferably contained in the 3' end of a second nucleic acid strand since the gene suppression activity of a double-stranded nucleic acid complex is improved.

In an embodiment, the modified internucleoside bond of a first nucleic acid strand and/or a second nucleic acid strand comprises a non-negatively charged (neutral or cationic respectively) internucleoside bond present in a neutral form or cationic form at certain pH (for example, human physiological pH (about 7.4), pH at a delivery site (for example, an organelle, a cell, a tissue, an organ, an organism), compared to anionic-form of the modified internucleoside bond (for example, -O-P(O)(O⁻)-O- (anionic-form of natural phosphate bond), - O-P(O)(S⁻)-O-(anionic-form of phosphorothioate bond)). In an embodiment, a modified internucleoside bond of a first nucleic acid strand and/or a second nucleic acid strand comprises a neutral internucleoside bond. In an embodiment, a modified internucleoside bond of a first nucleic acid strand and/or a second nucleic acid strand comprises a cationic internucleoside bond. In an embodiment, if a non-negatively charged internucleoside bond (for example, neutral internucleoside bond) is present in a neutral form, it does not have a moiety having pKa of less than 8, less than 9, less than 10, less than 11, less than 12, less than 13, or less than 14. In an embodiment, the non-negatively charged internucleoside bond is, for example, a methylphosphonate bond disclosed in U.S. Patent Nos. 5,264,423 and 5,286,717, a methylphosphotriester bond and ethylphosphotriester bond disclosed in U.S. Patent No. 5,955,599, a methoxypropylphosphonate bond disclosed in International Publication No. WO2015/168172, an internucleoside bond used in a self-neutralizing nucleic acid (ZON) disclosed in International Publication No. WO2016/081600. In an embodiment, a non-negatively charged internucleoside bond comprises a triazole moiety or an alkyne moiety. In an embodiment, a non-negatively charged internucleoside bond comprises a cyclic guanidine moiety and/or a guanidine moiety (preferably, TMG moiety) substituted with 1 to 4 C₁₋₆ alkyl groups. In an embodiment, a modified internucleoside bond containing a cyclic guanidine moiety has a partial structure represented by formula (III). In an embodiment, a guanidine moiety substituted with 1 to 4 C₁₋₆ alkyl groups has a partial structure represented by formula (IV). In an embodiment, a neutral internucleoside bond containing a cyclic guanidine moiety and/or a guanidine moiety substituted with 1 to 4 C₁₋₆ alkyl groups is chirally controlled. In an embodiment, the disclosure herein relates to a composition comprising an oligonucleotide that contains at least one neutral internucleoside bond and at least one phosphorothioate internucleoside bond. Without wishing to be bound by any particular theory, at least in some cases, a neutral internucleotide bond can improve properties and/or activity as compared to comparable nucleic acids that do not comprise a neutral internucleotide bond; for example, it can improve delivery, resistance to exonuclease and endonuclease, cellular uptake, endosome escape, and/or nuclear uptake.

In an embodiment, a second nucleic acid strand may be bound to a lipid. Examples of the lipid include, but are not limited to, tocopherol, cholesterol, fatty acid, phospholipid and an analog thereof; folic acid, vitamin C, vitamin B1, vitamin B2; estradiol, androstane and an analog thereof; steroid and an analog thereof; a ligand of LDLR, SRBI or LRP1/2; FK-506, and cyclosporine; and a lipid disclosed in PCT/JP2019/12077, PCT/JP2019/10392 and PCT/JP2020/035117.

"Tocopherol" is herein a methylated derivative of tocorol which is a liposoluble vitamin (vitamin E) having a cyclic structure called chroman. Tocorol has a strong antioxidant effect, and therefore functions *in vivo* as an antioxidant substance to scavenge free radicals produced by metabolism and protect cells from damage.

A plurality of different types of tocopherol are known based on the position of the methyl group bound to chroman comprising α-tocopherol, β-tocopherol, γ-tocopherol, and δ-tocopherol. A tocopherol herein may be any types of tocopherol. In addition, examples of the analog of tocopherol comprise various unsaturated analogs of tocopherol, such as α-tocotrienol, β-tocotrienol, γ-tocotrienol, and δ-tocotrienol. Preferably, tocopherol is α-tocopherol.

"Cholesterol" is herein a kind of sterol, also called steroid alcohol, which is especially abundant in animals. Cholesterol exerts an important function in the metabolic process *in vivo*, and in animal cells, it is also a major constituent of the membrane system of a cell, together with phospholipid. In addition, the cholesterol analog refers to various cholesterol metabolism products and their analogs, which are alcohols having a sterol backbone. Examples thereof include, but not limited to, cholestanol, lanosterol, cerebrosterol, dehydrocholesterol, and coprostanol.

An "analog" herein refers to a compound having a similar structure and property having the same or a similar basic backbone. The analog comprises, for example, a biosynthetic intermediate, a metabolism product, and a compound having a substituent. Those skilled in the art can determine whether or not a compound is an analog of another compound based on common general technical knowledge.

Cholesterol analogs are various cholesterol metabolites and analogs, which are alcohols having a sterol backbone. Examples of the analogs include, but are not limited to, cholestanol, lanosterol, cerebrosterol, dehydrocholesterol, and coprostanol.

In an embodiment, a second nucleic acid strand may be bound to tocopherol or cholesterol or an analog thereof. The second nucleic acid strand bound to cholesterol or an analog thereof may have a group represented by the following general formula (V): 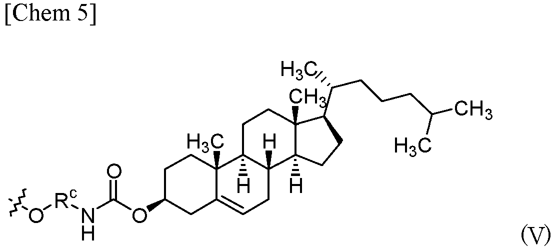 wherein R^{c} represents an alkylene group having 4 to 18 carbon atoms, preferably 5 to 16 carbon atoms optionally having a substituent (herein, the substituent is an alkyl group having 1 to 3 carbon atoms optionally substituted with a halogen atom or a hydroxy group, for example, a hydroxymethyl group, and carbon atoms not adjacent to each other in the alkylene group may be substituted with an oxygen atom).

R^{c} is not limited and it may be -(CH₂)₃-O-(CH₂)₂-O-(CH₂)₂-O-(CH₂)₂-O-(CH₂)₂-, - (CH₂)₃-O-(CH₂)₂-O-(CH₂)₂-O-(CH₂)₂-O-CH₂-CH (CH₂OH)-, or-(CH₂)₆-.

A group represented by the general formula (V) described above can be bound to the 5' end or 3' end of a second nucleic acid strand via a phosphate ester bond.

A lipid such as cholesterol or an analog thereof may be bound to any of the 5' end, 3' end, or both ends of a second nucleic acid strand. A lipid such as cholesterol or an analog thereof may be bound to a nucleotide in the interior part of a second nucleic acid strand. Although not limited, cholesterol or an analog thereof bound to the 5' end of a second nucleic acid strand is particularly suitable.

If a second nucleic acid strand contains a plurality of cholesterol or an analog thereof, they may be the same or different. For example, each of cholesterol and another cholesterol analog may be bound to the 5' end and 3' end of a second nucleic acid strand, respectively. As to the binding position, cholesterol and an analog thereof may be bound to a plurality of positions of a second nucleic acid strand, and/or a single position as a group. Cholesterol and an analog thereof may be bound to the 5' end and the 3' end of a second nucleic acid strand, respectively one by one.

A second nucleic acid strand and a lipid may be bound directly, or indirectly via a different substance.

When a second nucleic acid strand and a lipid are directly bound, the lipid may be bound to the second nucleic acid strand via, for example, a covalent bond, an ionic bond or a hydrogen bond. Considering that a more stable bond can be obtained, a covalent bond is preferable.

In an embodiment, a second nucleic acid strand is not bound to a ligand. The phrase "is not bound to a ligand" means that a lipid such as tocopherol or cholesterol is not bound. In another embodiment, the double-stranded nucleic acid complex of the present invention is not bound to a ligand: namely, neither a first nucleic acid strand nor a second nucleic acid strand is bound to a ligand.

When a second nucleic acid strand is indirectly bound to cholesterol or an analog thereof, they may be bound via a linking group (herein often referred to as a "linker"). The linker may be either a cleavable linker or an uncleavable linker.

The "cleavable linker" refers to a linker that can be cleaved under physiological conditions, for example, in a cell or in an animal body (e.g., in a human body). The cleavable linker is selectively cleaved with an endogenous enzyme such as nuclease. Examples of the cleavable linker include, but are not limited to, an amide, an ester, either one or both esters of a phosphodiester, a phosphate ester, a carbamate, and a disulfide bond, and a natural DNA linker. As an example, cholesterol or an analog thereof may be bound via a disulfide bond.

The "uncleavable linker " refers to a linker that cannot be cleaved under physiological conditions, for example, in a cell or in an animal body (e.g., in a human body). Examples of the uncleavable linker include, but are not limited to, a phosphorothioate bond, modified or unmodified deoxyribonucleosides linked by a phosphorothioate bond, or a linker consisting of modified or unmodified ribonucleosides. If a linker is a nucleic acid such as DNA or an oligonucleotide, the chain length, although not particularly limited, may be usually 2 to 20 base in length, 3 to 10 base in length, or 4 to 6 base in length.

An example of the linker is a linker represented by the following formula (VI). 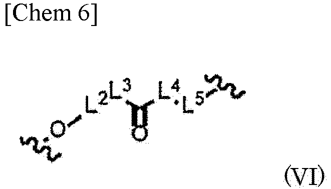 wherein L² represents a substituted or unsubstituted C₁-C₁₂ alkylene group (for example, propylene, hexylene, dodecylene), a substituted or unsubstituted C₃-C₈ cycloalkylene group (for example, cyclohexylene) -(CH₂)₂-O-(CH₂)₂-O-(CH₂)₂-O-(CH₂)₃-,-(CH₂)₂-O-(CH₂)₂-O-(CH₂)₂-O-(CH₂)₂-O-(CH₂)₃- or CH(CH₂-OH)-CH₂-O-(CH₂)₂-O-(CH₂)₂-O-(CH₂)₂-O-(CH₂)₃-, L³ represents -NH- or a bond, L⁴ represents a substituted or unsubstituted C₁-C₁₂ alkylene group (for example, ethylene, pentylene, heptylene, or undecylene), a substituted or unsubstituted C₃-C₈ cycloalkylene group (for example, cyclohexylene), -(CH₂)₂-[O-(CH₂)₂]ₘ-, or a bond, wherein m is an integer of 1 to 25, L⁵ represents-NH-(C=O)-, -(C=O)-, or a bond (herein, the substitution is preferably by a halogen atom).

In an embodiment, in a linker represented by formula (VI), L² is unsubstituted C₃-C₆ alkylene group (for example, propylene, hexylene), -(CH₂)₂-O-(CH₂)₂-O-(CH₂)₂-O-(CH₂)₃-, or -(CH₂)₂-O-(CH₂)₂-O-(CH₂)₂-O-(CH₂)₂-O-(CH₂)₃-, L³ is-NH-, and L⁴ and L⁵ are bonds.

Another example of the linker is a linker represented by the following formula (VII): 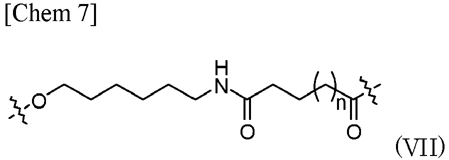 wherein n represents 0 or 1.

A first nucleic acid strand and/or a second nucleic acid strand (preferably a second nucleic acid strand) may further contain at least one functional moiety bound to a polynucleotide constituting a nucleic acid strand. A "functional moiety" refers to a moiety that provides a desired function to a double-stranded nucleic acid complex and/or a nucleic acid strand to which the functional moiety is bound. Examples of the desired function include a labeling function and a purification function. Examples of the moiety that provides a labeling function include compounds such as a fluorescent protein, and luciferase. Examples of the moiety that provides a purification function include compounds such as biotin, avidin, His-tag peptide, GST-tag peptide, and FLAG-tag peptide. In an embodiment, from the viewpoint that a first nucleic acid strand is highly specifically and efficiently delivered to a target site to extremely effectively suppress the expression of a target gene, it is preferable that a molecule having an activity to deliver a double-stranded nucleic acid complex according to an embodiment to a target site is bound to a second nucleic acid strand as a functional moiety. Examples of the moiety for providing a delivery function to a target include a lipid, an antibody, an aptamer, and a ligand to a specific receptor. In an embodiment, a first nucleic acid strand and/or a second nucleic acid strand (preferably a second nucleic acid strand) is bound to a functional moiety. The binding of a second nucleic acid and a functional moiety may be a direct binding or an indirect binding via another substance. In an embodiment, it is preferable that a second nucleic acid and a functional moiety are directly bound via, *e*.*g*., a covalent bond, an ionic bond, a hydrogen bond. A covalent bond is more preferable in view of stable binding.

In another embodiment, a second nucleic acid strand may further contain at least one overhang region at either one or both of the 5'-end side and 3'-end side of a complementary region. This embodiment is disclosed, for example, in International Publication No. WO2018/062510.

A first nucleic acid strand and a second nucleic acid strand may be bound via a linker. In this case, the first nucleic acid strand and the second nucleic acid strand can be linked via a linker to form a single strand. However, even in that case, the functional region has the same configuration as the double-stranded nucleic acid complex, and therefore such a single-stranded nucleic acid is also included herein as an embodiment of the double-stranded nucleic acid complex of the present invention. The linker can be any polymer. Examples of the polymer include a polynucleotide, polypeptide, and alkylene. Specifically, the polymer can be composed of a natural nucleotide such as DNA, and RNA, or a non-natural nucleotide such as a peptide nucleic acid and a morpholino nucleic acid. When a linker consists of a nucleic acid, the chain length of a linker may be at least 1 base, or 3 to 10 base or 4 to 6 base. A chain length is preferably 4 base. The position of the linker can be either on the 5' side or the 3' side of a first nucleic acid strand. For example, in the case of a configuration in which cholesterol or an analog thereof is bound to the 5' side of the second nucleic acid strand, the 5' end of the first nucleic acid strand and the 3' end of the second nucleic acid strand are to be linked via a linker. The linker may be cleavable or uncleavable.

In the double-stranded nucleic acid complex of the present invention, the antisense effect on the target transcription product provided by the first nucleic acid strand can be measured by a method commonly known in the art. For example, a double-stranded nucleic acid complex is introduced into, e.g., a cell, and then, a commonly known technique such as Northern blotting, quantitative PCR, or Western blotting is used to measure the effect. Whether the target gene expression is suppressed by a double-stranded nucleic acid complex or not can be determined by measuring the expression level of a target gene or the level of a target transcription product (*e*.*g*., the amount of mRNA, the amount of RNA such as microRNA, the amount of cDNA, and the amount of protein). As said criteria of the determination, although not limited, if a measured expression level of target gene or a measured level of a target transcription product decreases by at least 5%, at least 10%, at least 15%, at least 20%, at least 25%, at least 30%, or at least 40%, compared to that of a negative control (for example, vehicle administration), it may be determined that the double-stranded nucleic acid complex of the present invention produced an antisense effect.

Exemplary embodiments of the double-stranded nucleic acid complex of the present invention have been described above but the double-stranded nucleic acid complex of the present invention is not limited to the above exemplary embodiments.

### 1-4. Method for producing a double-stranded nucleic acid complex

The double-stranded nucleic acid complex of the present invention can be produced by those skilled in the art using an appropriate method commonly known in the art. Although not limited, the production is started first by designing and producing each of a first nucleic acid strand and a second nucleic acid strand constituting a double-stranded nucleic acid complex. For example, the first nucleic acid strand is designed based on the information on the base sequence of a target transcription product (*e*.*g*., the base sequence of a target gene), and then the second nucleic acid strand is designed as a complementary strand thereto. Subsequently, individual nucleic acid strands may be synthesized based on the information on the base sequences designed by use of a commercially available automatic nucleic acid synthesizer manufactured by, *e*.*g*., GE Healthcare, Thermo Fisher Scientific, or Beckman Coulter. Thereafter, the prepared oligonucleotides may be purified using, *e.g.*, a reverse-phase column.

In the case of a double-stranded nucleic acid complex to which a functional moiety is bound, the first nucleic acid strand may be produced in accordance with the above method. In contrast, a second nucleic acid strand to which a functional moiety is bound can be produced by subjecting a nucleic acid species to which a functional moiety is bound in advance to the synthesis and purification as mentioned above. For example, a second nucleic acid strand may be produced by performing the aforedescribed synthesis and purification using a nucleic acid species to which a functional moiety has been bound in advance. Alternatively, cholesterol or an analog thereof may be bound by a commonly known method to a second nucleic acid strand produced by performing the aforedescribed synthesis and purification. After individual nucleic acid strands are produced, the first nucleic acid strand and the second nucleic acid strand are annealed to produce a double-stranded nucleic acid complex to which a desired functional moiety is bound. Specifically, the nucleic acid is mixed in an appropriate buffer solution to denature it at about 90°C to 98°C for several minutes (*e*.*g*., 5 min), and then the nucleic acid is annealed at about 30°C to 70°C for about 1 to 8 hours to produce a double-stranded nucleic acid complex of the present invention. A method for linking a functional moiety to a nucleic acid is well known in the technical field. Further, a nucleic acid strand is available from various manufacturers (*e*.*g*., GeneDesign Inc.) by specifying the base sequence and the modification site and type.

### 1-5. Effect

According to the double-stranded nucleic acid complex of the present invention, the toxicity of a double-stranded nucleic acid complex can be reduced and eliminated without imparing the effectiveness of the double-stranded nucleic acid complex. Namely, liver toxicity and renal toxicity are reduced without impairing an antisense effect on a target gene, and induction of inflammation or gliosis or an abnormal increase of a cytokine or a chemokine is reduced, compared to conventional double-stranded nucleic acid complexes.

### 2. Pharmaceutical composition

### 2-1. Overview

A second aspect of the present invention relates to a pharmaceutical composition. The pharmaceutical composition of the present invention comprises the double-stranded nucleic acid complex according to said first aspect as an active ingredient. The pharmaceutical composition of the present invention has low liver toxicity and/or low renal toxicity. Furthermore, induction of inflammation or gliosis or an abnormal increase of a cytokine or a chemokine is reduced.

Each component that may be contained in the pharmaceutical composition of the present invention is specifically described below.

### 2-2. Configuration

### 2-2-1. Active ingredient

The pharmaceutical composition of the present invention encompasses at least a double-stranded nucleic acid complex according to the first aspect, as an active ingredient. The pharmaceutical composition of the present invention may comprise two or more types of double-stranded nucleic acid complexes.

The amount (content) of the double-stranded nucleic acid complex in a pharmaceutical composition varies depending on the type of double-stranded nucleic acid complex, the delivery site, the dosage form and dose of the pharmaceutical composition, and the type of carrier described below. Therefore, the content may be appropriately determined in consideration of individual conditions. Usually, the content may be controlled so that an effective amount of the double-stranded nucleic acid complex is contained in a single dose of the pharmaceutical composition. An "effective amount" refers to an amount that is required for the double-stranded nucleic acid complex to function as an active ingredient and produces little or no adverse side effects on the living body to which it is applied. The effective amount can vary depending on various conditions such as information on the subject, the administration route, and the frequency of administrations. The effective amount is finally determined by, *e*.*g*., a physician, a veterinarian, or a pharmacist. The "Information on the subject" is various information on the living body of an individual to which the pharmaceutical composition is to be applied. For example, if the subject is a human, examples of the information include age, body weight, gender, dietary habit, health status, stage of progression or grade of severity of the disease, drug sensitivity, and whether a combined drug is used or not.

### 2-2-2. Carrier

The pharmaceutical composition of the present invention may comprise a pharmaceutically acceptable carrier. A "pharmaceutically acceptable carrier" refers to an additive commonly used in the field of pharmaceutical preparation. Examples of the carrier include a solvent, a vegetable oil, a base, an emulsifier, a suspending agent, a surfactant, a pH adjuster, a stabilizer, a seasoning, a flavor, an excipient, a vehicle, a preservative, a binder, a diluent, an isotonizing agent, a sedative, a bulking agent, a disintegrating agent, a buffering agent, a coating agent, a lubricant, a colorant, a sweetener, a thickener, a masking agent, and a dissolution aid and other additives.

The solvent may be any of, for example, water or other pharmaceutically acceptable aqueous solutions, and a pharmaceutically acceptable organic solvent. Examples of the aqueous solution include a physiological saline, an isotonic solution containing glucose or an additive, a phosphate buffer, and a sodium acetate buffer. Examples of the additive include D-sorbitol, D-mannose, D-mannitol, sodium chloride, and in addition, low-concentration nonionic surfactant and polyoxyethylene sorbitan fatty acid ester.

The carrier mentioned above is used for not only avoiding or suppressing *in-vivo* enzymatic degradation of a double-stranded nucleic acid complex serving as an active ingredient but also facilitating formulation or administration and maintaining the dosage form and drug efficacy. An appropriate carrier may be used according to the needs.

### 2-2-3. Dosage form

The dosage form of the pharmaceutical composition of the present invention is not particularly limited as long as it can be delivered to a target site without inactivating an active ingredient, *i*.*e*., a double-stranded nucleic acid complex according to the first aspect, by, *e.g.*, degradation, and can produce the pharmacological effect of the active ingredient *in vivo* (antisense effect on the expression of a target gene).

The specific dosage form varies depending on the administration method and/or formulation condition. The administration methods can be roughly classified into parenteral administration and oral administration. The dosage form may be appropriately determined according to the individual administration methods.

If parenteral administration is employed as the administration method, the dosage form is preferably a liquid, which enables direct administration to a target site, or systemic administration through the circulatory system. Examples of the liquid include an injectable solution. The injectable solution can be used appropriately in combination with, *e*.*g*., said excipient, elixir, emulsifier, suspending agent, surfactant, stabilizer and/or pH adjuster and mixed in accordance with pharmaceutical practices generally approved to formulate in unit doses. Other than the liquid, an ointment, a plaster, a cataplasm, a transdermal patch, a lotion, an inhalant, an aerosol, an eye drop, and a suppository may be employed.

If oral administration is employed as the administration method, a solid preparation or a liquid may be preferably used as the dosage form. Examples of the dosage form include a tablet, a capsule, a drop, and lozenge, a granule, a pill, a dusting powder, a powder, and a liquid medication, an emulsion, a syrup, a pellet, a sublingual formulation, a peptizer, a buccal, a paste, a suspension, an elixir, a coating agent, an ointment, a plaster, a cataplasm, a transdermal agent, a lotion, an inhalant, an aerosol an eye drop, an injection, and a suppository. In the case of the solid preparation, if necessary, film-coating forms commonly known in the technical field can be used. Examples of the film-coating forms include a sugar-coated tablet, a gelatin-coated tablet, an enteric-coated tablet, a film-coated tablet, a double-layer tablet, and a multilayer tablet.

The specific shape and size of each of the dosage forms mentioned above are not particularly limited, as long as they fall within the ranges of dosage forms commonly known in the technical field. The drug product formulation of the pharmaceutical composition of the present invention may be performed in accordance with a method commonly known in the technical field.

In a certain embodiment, the double-stranded nucleic acid complex of the present invention has excellent solubility in water, the 2nd fluid according to the dissolution test of the Japanese Pharmacopoeia, or the 2nd fluid according to the disintegration test of Japanese Pharmacopoeia, excellent *in vivo* kinetics (*e*.*g*., serum drug half-life, intracerebral transfer, metabolic stability, CYP inhibition), low toxicity (*e*.*g*., acute toxicity, chronic toxicity, genetic toxicity, reproductive toxicity, cardiotoxicity, drug interaction, carcinogenicity, phototoxicity, excellent as a medical drug), and fewer side effects (*e*.*g*., inhibition of sedation, avoidance of layered necrosis). Thus, the double-stranded nucleic acid complex of the present invention has properties excellent as a medical product.

### 2-3. Dosage form and dose

The preferable dosage form of a pharmaceutical composition is not particularly limited herein. For example, the dosage form may be oral administration or parenteral administration. Examples of the parenteral administration include intramuscular administration, intravenous administration, intra-arterial administration, intraperitoneal administration, subcutaneous administration (including continuous subcutaneous administration by implant), intradermal administration, trachea/bronchial administration, rectal administration, administration by blood transfusion, intraventricular administration, intrathecal administration, nasal administration, and intramuscular administration.

When a pharmaceutical composition is applied by administration or ingestion, the dose or ingestion dose may be, for example, from 0.00001 mg/kg/day to 10000 mg/kg/day, or from 0.001 mg/kg/day to 100 mg/kg/day in terms of the double-stranded nucleic acid complex contained in the pharmaceutical composition. A pharmaceutical composition may be administered in a single dose or multiple dose. In the multiple dose, a pharmaceutical composition can be administered daily or at appropriate time intervals (*e*.*g*., at intervals of one day, 2 days, 3 days, 1 week, 2 weeks, or one month), *e*.*g*., 2 to 20 times. The dose of the double-stranded nucleic acid complex per time may be, for example, 0.001 mg/kg or more, 0.005 mg/kg or more, 0.01 mg/kg or more, 0.25 mg/kg or more, 0.5 mg/kg or more, 1.0 mg/kg or more, 2.0 mg/kg or more, 2.5 mg/kg or more, 3.0 mg/kg or more, 4.0 mg/kg or more, 5 mg/kg or more, 10 mg/kg or more, 20 mg/kg or more, 30 mg/kg or more, 40 mg/kg or more, 50 mg/kg or more, 75 mg/kg or more, 100 mg/kg or more, 150 mg/kg or more, 200 mg/kg or more, 300 mg/kg or more, 400 mg/kg or more, or 500 mg/kg or more. For example, the dose can be appropriately selected from the range of from 0.001 mg/kg to 500 mg/kg (*e*.*g*., 0.001 mg/kg, 0.01 mg/kg, 0.1 mg/kg, 1 mg/kg, 5 mg/kg, 10 mg/kg, 50 mg/kg, 100 mg/kg, or 200 mg/kg).

The double-stranded nucleic acid complex of the present invention may be administered at a dose of from 0.01 to 10 mg/kg (*e*.*g*., about 6.25 mg/kg) and at a frequency of twice a week, for four times. Further, the double-stranded nucleic acid complex may be administered at a dose of 0.05 to 30 mg/kg (*e*.*g*., about 25 mg/kg) and at a frequency of once or twice a week for two to four times, for example, at a frequency of twice a week for two times. If such a dosing regimen (divided administration) is employed, the toxicity can be lowered (*e*.*g*., platelet reduction is avoided) and load to a subject can be reduced compared to a higher-dose single administration.

Even if the pharmaceutical composition is repeatedly administered, a suppression effect can be produced in an additive manner in a cell. Furthermore, in the case of repeated administration, it is better to administer the pharmaceutical composition at some intervals (*e*.*g*., half a day or longer) to improve the efficacy.

In an embodiment, the pharmaceutical composition of the present aspect is intraventricularly or intrathecally administered. When the pharmaceutical composition of this aspect is intraventricularly or intrathecally administered to monkeys and humans, the dose may be 0.01 mg or more, 0.1 mg or more, or 1 mg or more, for example, 2 mg or more, 3 mg or more, 4 mg or more, 5 mg or more, 10 mg or more, 20 mg or more, 30 mg or more, 40 mg or more, 50 mg or more, 75 mg or more, 100 mg or more, 200 mg or more, 300 mg or more, 400 mg or more, or 500 mg or more, that is, 0.01 mg to 1000 mg, 0.1 mg to 200 mg, or 1 mg to 20 mg. In the case of mice, the dose may be 1 µg or more.

In an embodiment, the pharmaceutical composition of the present aspect is intravenously or subcutaneously administered. When the pharmaceutical composition of this aspect is intravenously or subcutaneously administered, the dose may be 0.01 mg/kg or more, 0.1 mg/kg or more, or 1 mg/kg or more, for example, 2 mg/kg or more, 3 mg/kg or more, 4 mg/kg or more, 5 mg/kg or more, 10 mg/kg or more, 20 mg/kg or more, 30 mg/kg or more, 40 mg/kg or more, 50 mg/kg or more, 75 mg/kg or more, 100 mg/kg or more, 150 mg/kg or more, 200 mg/kg or more, 300 mg/kg or more, 400 mg/kg or more, or 500 mg/kg or more, that is, 0.01 mg/kg to 1000 mg/kg, 0.1 mg/kg to 100 mg/kg, or 1 mg/kg to 10 mg/kg.

### 2-4. Disease

The disease to which a pharmaceutical composition is applied is not limited. The pharmaceutical composition can be applied to diseases associated with an increased expression of a target gene, such as a neurological disease, a central nervous system disease, a metabolic disease, a tumor and an infection. In an embodiment, the pharmaceutical composition according to this aspect can be used for treating a central nervous system disease in a subject. Examples of the central nervous system disease to which the pharmaceutical composition of this aspect is applied include, but are not limited to, brain tumor, Alzheimer's disease, Parkinson's disease, amyotrophic lateral sclerosis, multiple sclerosis, and Huntington's disease.

### 2-5. Effect

The pharmaceutical composition of the present invention has low liver toxicity and/or low renal toxicity. Furthermore, the pharmaceutical composition of the present invention reduces induction of inflammation or gliosis or an abnormal increase of a cytokine or a chemokine by *e*.*g*., intraventricular administration, intrathecal administration, intravenous administration or subcutaneous administration.

According to the pharmaceutical composition of the present invention, a disease can be treated or prevented by suppressing or inhibiting the expression of a particular gene substantially without liver toxicity and/or renal toxicity. Also, a disease can be treated or prevented while reducing an abnormal increase of a cytokine or a chemokine without substantial induction of inflammation or gliosis.

In particular, since a high-dose administration of a nucleic acid agent is required for treating a neurological disease such as Alzheimer's disease, a side effect such as serious liver disorder is problematic. However, according to the pharmaceutical composition of the present invention, it is possible to significantly reduce such a side effect.

The present invention also provides a method for treating and/or preventing a disease such as central nervous system disease, comprising administering the double-stranded nucleic acid complex or the pharmaceutical composition mentioned above to a subject.

### Examples

Hereinafter, the present invention is described further specifically with reference to Examples. However, the technical scope of the present invention is not limited to these Examples.

### <Example 1: Gene suppression effect and toxicity-reducing effect by intraventricular administration of HDO (scpBNA)>

### (Purpose)

The gene suppression effect and toxicity-reducing effect by intraventricular administration are investigated by *in vivo* experiments for heteroduplex oligonucleotides comprising a first nucleic acid strand consisting of a scpBNA/DNA gapmer antisense nucleic acid (hereinafter referred to as "ASO (scpBNA)") and a second nucleic acid strand composed of RNA having a complementary base sequence to the first nucleic acid strand (hereinafter referred to as "HDO (scpBNA)").

### (Method)

### (1) Preparation of nucleic acid

The base sequences of the first nucleic acid strand and the second nucleic acid strand constituting HDO (scpBNA) and chemical modifications thereof used in this Example are shown in Table 1 and Figure 3D.

**[Table 1]**

| | | Sequence (5'-3') | SEQ ID NO: |
|---|---|---|---|
| HDO (scpBNA) | First nucleic acid strand: ASO(scpBNA) | A(B)^T(B)^A(B)^g^t^c^a^c^t^c^t^g^g^ T(B)^G(B)^A(B) | 1 |
| | Second nucleic acid strand: cRNA | **U^C^ACCAGAGUGACU^A^U** | 2 |

| | | | |
|---|---|---|---|
| Lower case letter: DNA; Upper case letter: RNA; Upper case letter (B): scpBNA; ^: phosphorothioate bond (PS bond) | | | |

The first nucleic acid strand of HDO (scpBNA) is a scpBNA/DNA gapmer antisense nucleic acid comprising a 5'-wing region and a 3'-wing region each consisting of 3 scpBNA nucleosides at the 5' end and the 3' end, respectively, with 10 DNA nucleosides between them. The base sequence of the first nucleic acid strand is complementary to a part of mouse microtubule-associated protein tau (Mapt) mRNA. The second nucleic acid strand of HDO (scpBNA) is composed of RNA having a complementary sequence to the first nucleic acid strand. It is noted that neither the first nucleic acid strand nor second nucleic acid strand of HDO (scpBNA) is bound to a ligand.

It is noted that the scpBNA used in Examples herein is a non-natural nucleoside represented by the following formula (I):

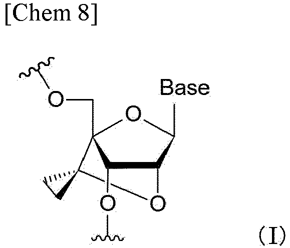

As a comparative control of the HDO (scpBNA), a heteroduplex oligonucleotide comprising an LNA/DNA gapmer antisense nucleic acid (hereinafter referred to as "ASO (LNA)") was used (hereinafter referred to as "HDO (LNA)"). The structures and base sequences of the first nucleic acid strand and the second nucleic acid strand constituting HDO (LNA) used in this Example are shown in Table 2 and Figure 3B. It is noted that neither the first nucleic acid strand nor second nucleic acid strand of HDO (LNA) is bound to a ligand.

**[Table 2]**

| | | Sequence (5'-3') | SEQ ID NO: |
|---|---|---|---|
| HDO (LNA) | First nucleic acid strand: ASO(LNA) | A(L)^T(L)^A(L)^g^t^c^a^c^t^c^t^g^g^ T(L)^G(L)^A(L) | 3 |
| | Second nucleic acid strand: cRNA | **U^C^ACCAGAGUGACU^A^U** | 2 |

| | | | |
|---|---|---|---|
| Lower case letter: DNA; Upper case letter: RNA; Upper case letter (L): LNA; ^: phosphorothioate bond (PS bond) | | | |

In order to prepare the double-stranded nucleic acid complex described above, equimolar amounts of the first nucleic acid strand and the second nucleic acid strand were mixed, followed by heating the solution at 95°C for 5 minutes, cooling it to 37°C and keeping it for one hour, thereby annealing the nucleic acid strands to prepare the double-stranded nucleic acid complex. The nucleic acids annealed were stored at 4°C or on ice. Synthesis of all oligonucleotides was outsourced to Gene Design (Osaka, Japan).

Further, in this Example, ASO (scpBNA) consisting only of the first nucleic acid strand shown in Table 1, and ASO (LNA) consisting only of the first nucleic acid strand shown in Table 2, were also subjected to the following experiments as single stranded gapmer antisense nucleic acids.

### (2) In vivo experiment

Seven week-old female ICR mice were fixed in a stereotaxic apparatus under 2.5 to 4% isoflurane anesthesia. Thereafter, the skin between the ears was dissected at a length of 2 to 3 cm in the direction from front to back and perforated at a position on the 1 mm-left side and 0.2 mm-back side of the bregma by using a 1 mm-diameter drill. A Hamilton syringe was filled with a nucleic acid agent. The needle was inserted at a depth of about 3 mm from the perforated site and the nucleic acid agent was administered to the left ventricle (n = 2) at a rate of 2 to 3 µl/minute and a dose of 24 µmol per mouse. Then, the skin was sutured with a nylon thread. Day 7 after injection, PBS was perfused in the mice. Thereafter the mice were dissected and the left hippocampus was isolated.

### (3) Analysis of expression

RNA was extracted with IsogenI kit (Gene Design) from the isolated left hippocampus, and cDNA was synthesized with Transcriptor Universal cDNA Master, DNase (Roche Diagnostics) in accordance with the protocol.

Subsequently, for evaluating the gene suppression effect and toxicity of various nucleic acid agents, quantitative RT-PCR was performed using the cDNA obtained as a template. The quantitative RT-PCR was performed by TaqMan (Roche Applied Science). As the primers used in the quantitative RT-PCR, a product designed and produced by Thermo Fisher Scientific (old name: Life Technologies Corp.) based on various number of genes, was used. The amplification conditions (temperature and time) were as follows: a cycle consisting of 95°C for 15 seconds, 60°C for 30 seconds, and 72°C for one second was repeated 40 times.

For evaluating the gene suppression effect by the nucleic acid agent, the expression levels of Mapt mRNA and Actb mRNA (internal standard gene) were measured and the ratio of the expression levels of the two mRNAs was calculated. Also, for evaluating the toxicity of the nucleic acid agents, the expression level of Tumor Necrosis Factor-α (TNFα) mRNA was measured as the index of inflammatory cytokines, and the expression level of glial fibrillary acidic protein (GFAP) mRNA was measured as the index of astrocyte activation/gliosis, in a similar manner.

### (Results)

Figure 4 shows the Mapt mRNA expression levels in the left hippocampus of mice in which various nucleic acid agents were administered to the left ventricle. A significant difference was not observed in the Mapt gene suppression effect in the intraventricular administration of ASO (LNA), HDO (LNA), ASO (scpBNA), and HDO (scpBNA).

Figure 5 shows the TNFα mRNA and GFAP mRNA expression levels in the left hippocampus of mice in which various nucleic acid agents were administered to the left ventricle. In ASO (LNA) and HDO (LNA), a significant increase in the expression level of TNFα mRNA and GFAP mRNA was observed compared to a PBS administration case. In contrast, in the cases of ASO (scpBNA) and HDO (scpBNA), a significant increase in the expression level of TNFα mRNA and GFAP mRNA was not observed compared to that of the PBS administration, indicating that the induction of inflammatory cytokines/gliosis was reduced.

From these results, it was demonstrated that scpBNA has an effect of reducing side effects in the central nervous system without attenuating the gene regulation effect that conventional LNA has. The reduction effect was observed not only for a single-strand ASO but also for HDO structure at a similar level.

### <Example 2: Gene suppression effect and liver toxicity-reducing effect by single-dose intravenous administration of Toc-HDO (scpBNA) targeting mMalat1>

### (Purpose)

The target gene expression suppression effect as well as toxicity-reducing effect by single-dose intravenous administration are investigated for a double-stranded nucleic acid complex consisting of a scpBNA/DNA gapmer antisense nucleic acid and a tocopherol-bound complementary strand and a double-stranded nucleic acid complex consisting of an AmNA/DNA gapmer antisense nucleic acid and a tocopherol-bound complementary strand which target mMalat1.

### (Method)

### (1) Preparation of nucleic acid

Metastasis-associated lung adenocarcinoma transcription product 1 (mMalat1) was used as a target gene. The base sequences of the first nucleic acid strands and second nucleic acid strands of 3 types of double-stranded nucleic acid complexes used in this Example are shown in Table 3 and Figure 6.

**[Table 3]**

| | | Sequence (5'-3') | SEQ ID NO: |
|---|---|---|---|
| Toc-HDO (LNA) | First nucleic acid strand | **G(L)^T(L)^T(L)^c^a^c^t^g^a^a^t^G(L)^C(L)** | 4 |
| | Second nucleic acid strand | **Toc-G(M)^C(M)^AUUCAGUGA^A(M)^C(M)** | 5 |
| Toc-HDO (scpBNA) | First nucleic acid strand | **G(L)^T(B)^T(B)^c^a^c^t^g^a^a^t^G(L)^C(B)** | 6 |
| | Second nucleic acid strand | **Toc-G(M)^C(M)^AUUCAGUGA^A(M)^C(M)** | 5 |
| Toc-HDO (AmNA) | First nucleic acid strand | **G(L)^T(N)^T(N)^c^a^c^t^g^a^a^t^G(L)^C(N)** | 7 |
| | Second nucleic acid strand | **Toc-G(M)^C(M)^AUUCAGUGA^A(M)^C(M)** | 5 |

| | | | |
|---|---|---|---|
| Lower case letter: DNA; Upper case letter: RNA; Upper case letter (L): LNA (C(L) represents 5-methylcytosine LNA); Upper case letter (B): scpBNA (C(B) represents 5-methylcytosine scpBNA); Upper case letter (N): AmNA (C(N) represents 5-methylcytosine AmNA); Upper case letter (M): 2'-OMe RNA; ^: phosphorothioate bond (PS bond); Toc: tocopherol | | | |

The first nucleic acid strands described above target mMalat1 gene and are composed of a 13mer gapmer having a base sequence complementary to a part of the non-coding RNA of malat1 as a transcription product thereof. The first nucleic acid strand of Toc-HDO (LNA) consists of a 5'-wing region composed of 3 LNA nucleosides at the 5' end, a 3'-wing region composed of 2 LNA nucleosides at the 3' end, and 8 DNA nucleosides between them. In Toc-HDO (scpBNA) and Toc-HDO (AmNA), a part of the LNA nucleosides in the 5' and 3'-wing regions were substituted with a scpBNA or AmNA nucleoside.

On the other hand, the second nucleic acid strands are composed of a tocopherol-bound complementary strand RNA having a sequence complementary to the first nucleic acid strand and tocopherol bound at the 5' end thereof.

It is noted that AmNA used in the Examples herein is a non-natural nucleoside represented by the following formula (II): 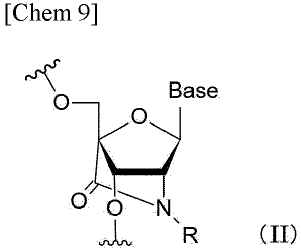 wherein R represents a methyl group.

The double-stranded nucleic acid complexes were prepared by the same method as in Example 1 (1).

### (2) In vivo experiment

As the mice to which a double-stranded nucleic acid complex agent was to be administered, 6 to 7-week-old male C57BL/6 mice having a weight of 20 g were used. Four mice were used for each condition.

The double-stranded nucleic acid complex agent was intravenously injected to mice in a single dose of 50 mg/kg through the tail vein. Mice used as a negative control group were also prepared by injecting PBS alone in a single dose. At the time point of 72 hours after administration, blood was sampled and PBS was perfused in the mice. Thereafter, the mice were dissected and the liver, kidney, cardiac muscle, and musculus quadriceps femoris were isolated.

### (3) Expression analysis

Using a high-throughput fully automated nucleic acid extractor MagNA Pure 96 (Roche Life Science), mRNA was extracted from individual tissues in accordance with the protocol. In accordance with the protocol of Transcriptor Universal cDNA Master (Roche Life Science), cDNA was synthesized. Quantitative RT-PCR was performed using TaqMan (Roche Life Science). As the primers used in the quantitative RT-PCR, a product designed and produced by Thermo Fisher Scientific based on various number of genes was used. The PCR conditions (temperature and time) were as follows: a cycle consisting of 95 °C for 15 seconds, 60°C for 30 seconds, and 72°C for one second was repeated 40 times. Amplified products obtained were quantified by quantitative RT-PCR and the expression levels of malat1 mRNA and GAPDH mRNA (internal standard gene) were calculated. The ratio of the expression levels of both mRNAs was calculated to obtain a relative expression level. Then, mean values and standard errors of the relative expression levels were calculated. The differences between individual groups were analyzed by t-test.

### (4) Serum analysis

The serum obtained by blood sampling were analyzed by outsourcing to SRL Hachioji Lab.

### (Results)

The results of the expression analysis are shown in Figures 7 and 8. Toc-HDO (scpBNA) and Toc-HDO (AmNA) exhibited comparable gene suppression effects as those of Toc-HDO (LNA) in all of the liver (Figure 7A), the kidney (Figure 7B), the musculus quadriceps femoris (Figure 8A), and the cardiac muscle (Figure 8B).

The results of the serum analysis are shown in Figure 9A. In the Toc-HDO (LNA) administration group, the measured values of AST (aspartate aminotransferase) and ALT (alanine aminotransferase) significantly increased, indicating reduction in the liver function. In contrast, in the Toc-HDO (scpBNA) administration group and Toc-HDO (AmNA) administration group, no significant increases in AST and ALT were detected, indicating that liver toxicity was reduced.

Further, in the Toc-HDO (LNA) administration group, a reduction in the body weight was observed, whereas, in the Toc-HDO (scpBNA) administration group and the Toc-HDO (AmNA) administration group, a significant change in the body weight was not observed (Figure 9B).

From the results above, it was demonstrated that the toxicity of HDO can be reduced without reducing a gene suppression effect of HDO by substituting a part of LNA with scpBNA or AmNA in the wing regions of a first nucleic acid strand.

### <Example 3: Gene suppression effect and liver toxicity-reducing effect by single-dose intravenous administration of Toc-HDO (scpBNA) targeting PTEN>

### (Purpose)

The target gene-expression suppression effect and toxicity-reducing effect by single-dose intravenous administration are investigated for a double-stranded nucleic acid complex consisting of a scpBNA/DNA gapmer antisense nucleic acid and a tocopherol-bound complementary strand and a double-stranded nucleic acid complex consisting of an AmNA/DNA gapmer antisense nucleic acid and a tocopherol-bound complementary strand which target PTEN.

### (Method)

PTEN (Phosphatase and Tensin Homolog Deleted from Chromosome 10) was used as a target gene. The base sequences of first nucleic acid strands and second nucleic acid strands and chemical modifications of 3 types of double-stranded nucleic acid complexes used in this Example are shown in Table 4 and Figure 10.

**[Table 4]**

| | | Sequence (5'-3') | SEQ ID NO: |
|---|---|---|---|
| Toc-HDO (LNA) | First nucleic acid strand | **T(L)^C(L)^a^t^g^g^c^t^g^c^a^g^C(L)^T(L)** | 8 |
| | Second nucleic acid strand | **Toc-A(M)^G(M)^CUGCAGCCAU^G(M)^A(M)** | 9 |
| Toc-HDO (scpBNA) | First nucleic acid strand | **T(B)^C(B)^a^t^g^g^c^t^g^c^a^C(B)^T(B)** | 10 |
| | Second nucleic acid strand | **Toc-A(M)^G(M)^CUGCAGCCAU^G(M)^A(M)** | 9 |
| Toc-HDO (AmNA) | First nucleic acid strand | **T(N)^C(N)^a^t^g^g^c^t^g^c^a^g^C(N)^T(N)** | 11 |
| | Second nucleic acid strand | **Toc-A(M)^G(M)^CUGCAGCCAU^G(M)^A(M)** | 9 |

| | | | |
|---|---|---|---|
| Lower case letter: DNA; Upper case letter: RNA; Upper case letter (L): LNA (C(L) represents 5-methylcytosine LNA); Upper case letter (B): scpBNA (C(B) represents 5-methylcytosine scpBNA); Upper case letter (N): AmNA (C(N) represents 5-methylcytosine AmNA); Upper case letter (M): 2'-OMe RNA; ^: phosphorothioate bond (PS bond); Toe: tocopherol | | | |

The first nucleic acid strands described above target PTEN gene and is composed of 14mer gapmer having a base sequence complementary to a part of PTEN mRNA. The first nucleic acid strand of Toc-HDO (LNA) consists of a 5'-wing region which is composed of two LNA nucleosides at the 5' end, a 3'-wing region which is composed of 2 LNA nucleosides at the 3' end, and 10 DNA nucleosides between them. In Toc-HDO (scpBNA) and Toc-HDO (AmNA), the whole LNA nucleosides in the 5' and 3'-wing regions are substituted with scpBNA or AmNA nucleosides.

On the other hand, a second nucleic acid strand is composed of a tocopherol-bound complementary strand RNA having a sequence complementary to the first nucleic acid strand and tocopherol bound at the 5' end thereof.

The double-stranded nucleic acid complexes were prepared by the same method as in Example 1 (1).

*In vivo* experiment was performed in the same manner as in Example 2 except that a double-stranded nucleic acid complex agent was intravenously injected to mice at a dose of 35 mg/kg. The expression analysis and serum analysis were performed by the same method as in Example 2.

### (Results)

The results of the expression analysis are shown in Figures 11 and 12. Toc-HDO (scpBNA) and Toc-HDO (AmNA) mostly exhibited gene suppression effects which were comparable with or more than those of Toc-HDO (LNA) in the liver (Figure 11A), the kidney (Figure 11B), the musculus quadriceps femoris (Figure 12A), and the cardiac muscle (Figure 12B).

The results of the serum analysis are shown in Figure 13A. In the Toc-HDO (LNA) administration group, AST and ALT significantly increased, indicating reduction of the liver function. In contrast, in the Toc-HDO (scpBNA) administration group and Toc-HDO (AmNA) administration group, no significant increases in AST and ALT were detected, indicating reduction of liver toxicity.

Further, a significant reduction in the body weight was observed in the Toc-HDO (LNA) administration group, whereas a significant change in the body weight was not observed in the Toc-HDO (scpBNA) administration group and the Toc-HDO (AmNA) administration group (Figure 13B).

From the results described above, it was demonstrated that the toxicity of HDO can be reduced without reducing a gene suppression effect of HDO by substituting the whole or a part of the nucleosides constituting the wing regions in the first nucleic acid strand with scpBNA or AmNA.

<Example 4: Gene suppression effect and liver toxicity-reducing effect by single-dose intravenous administration of Toc-HDO (scpBNA) targeting SR - B 1 >

### (Purpose)

The target gene expression suppression effect and toxicity-reducing effect by a single-dose intravenous administration were investigated for a double-stranded nucleic acid complex consisting of a scpBNA/DNA gapmer antisense nucleic acid and a tocopherol-bound complementary strand and a double-stranded nucleic acid complex consisting of an AmNA/DNA gapmer antisense nucleic acid and a tocopherol-bound complementary strand which target SR-B 1.

### (Method)

Scavenger receptor B 1 (scavenger receptor B 1; SR-B1) gene was used as a target gene. The base sequences of the first nucleic acid strand and the second nucleic acid strand of 3 types of double-stranded nucleic acid complexes used in this Example are shown in Table 5 and Figure 10.

**[Table 5]**

| | | Sequence (5'-3') | SEQ ID NO: |
|---|---|---|---|
| Toc-HDO (LNA) | First nucleic acid strand | **T(L)^C(L)^a^g^t^c^a^t^g^a^c^t^T(L)^C(L)** | 12 |
| | Second nucleic acid strand | **Toc-G(M)^A(M)^AGUCAUGACU^G(M)^A(M)** | 13 |
| Toc-HDO (scpBNA) | First nucleic acid strand | **T(B)^C(B)^a^g^t^c^a^t^g^a^c^t^T(B)^C(B)** | 14 |
| | Second nucleic acid strand | **Toc-G(M)^A(M)^AGUCAUGACU^G(M)^A(M)** | 13 |
| Toc-HDO (AmNA) | First nucleic acid strand | **T(N)^C(N)^a^g^t^c^a^t^g^a^c^t^T(N)^C(N)** | 15 |
| | Second nucleic acid strand | **Toc-G(M)^A(M)^AGUCAUGACU^G(M)^A(M)** | 13 |

| | | | |
|---|---|---|---|
| Lower case letter: DNA; Upper case letter: RNA; Upper case letter (L): LNA (C(L) represents 5-methylcytosine LNA); Upper case letter (B): scpBNA (C(B) represents 5-methylcytosine scpBNA); Upper case letter (N): AmNA (C(N) represents 5-methylcytosine AmNA); Upper case letter (M): 2'-OMe RNA; ^: phosphorothioate bond (PS bond); Toe: tocopherol | | | |

The first nucleic acid strands described above target SR-B1 gene and are composed of a 14mer gapmer having a base sequence complementary to a part of SR-B1 mRNA. The first nucleic acid strand of Toc-HDO (LNA) consists of a 5'-wing region composed of 2 LNA nucleosides at the 5' end, a 3'-wing region composed of 2 LNA nucleosides at the 3' end, and 10 DNA nucleosides between them. In Toc-HDO (scpBNA) and Toc-HDO (AmNA), the whole LNA nucleosides in the 5' and 3'-wing regions are substituted with scpBNA or AmNA nucleosides.

On the other hand, the second nucleic acid strands are composed of a tocopherol-bound complementary strand RNA having a sequence complementary to the first nucleic acid strand and tocopherol bound at the 5' end thereof.

The double-stranded nucleic acid complexes were prepared by the same method as in Example 1 (1).

An *in-vivo* experiment, expression analysis, and serum analysis were performed by the same method as in Example 2.

### (Results)

The results of the expression analysis are shown in Figures 14 and 15. Toc-HDO (scpBNA) and Toc-HDO (AmNA) exhibited comparable gene suppression effects as those of Toc-HDO (LNA) in all of the liver (Figure 14A), the kidney (Figure 14B), the musculus quadriceps femoris (Figure 15A), and the cardiac muscle (Figure 15B).

The results of the serum analysis are shown in Figure 16A. In the Toc-HDO (LNA) administration group, AST and ALT significantly increased, indicating reduction of liver function. In contrast, in the Toc-HDO (scpBNA) administration group and Toc-HDO (AmNA) administration group, AST and ALT did not increase, indicating that liver toxicity was not detected.

Further, a reduction in the body weight was observed in the Toc-HDO (LNA) administration group, whereas no significant change in body weight was observed in the Toc-HDO (scpBNA) administration group and the Toc-HDO (AmNA) administration group (Figure 16B).

From the results described above, it was demonstrated that the toxicity of HDO can be reduced without reducing a gene suppression effect of HDO by substituting the whole or a part of the nucleosides constituting the wing regions of a first nucleic acid strand with scpBNA or AmNA.

### <Example 5: Gene suppression effect and liver toxicity-reducing effect by multiple-dose intravenous administration of Chol-HDO (scpBNA)>

### (Purpose)

The target gene expression suppression effect as well as toxicity-reducing effect by multiple-dose intravenous administration were investigated for a double-stranded nucleic acid complex consisting of a scpBNA/DNA gapmer antisense nucleic acid and a cholesterol-bound complementary strand which targets SR-B 1.

### (Method)

### (1) Preparation of nucleic acid

SR-B 1 gene was used as a target gene. The base sequences of first nucleic acid strands and second nucleic acid strands of 2 types of double-stranded nucleic acid complexes used in this Example are shown in Table 6 and Figure 17.

**[Table 6]**

| | | Sequence (5'-3') | SEQ ID NO: |
|---|---|---|---|
| Chol-HDO (LNA) | First nucleic acid strand | **T(L)^C(L)^a^g^t^c^a^t^g^a^c^t^T(L)^C(L)** | 16 |
| | Second nucleic acid strand | **Chol-G(M)^A(M)^AGUCAUGACU^G(M)^A(M)** | 17 |
| Chol-HDO (scpBNA) | First nucleic acid strand | **T(B)^C(B)^a^g^t^c^a^t^g^a^c^t^T(B)^C(B)** | 18 |
| | Second nucleic acid strand | **Chol-G(M)^A(M)^AGUCAUGACU^G(M)^A(M)** | 17 |

| | | | |
|---|---|---|---|
| Lower case letter: DNA; Upper case letter: RNA; Upper case letter (L): LNA (C(L) represents 5-methylcytosine LNA); Upper case letter (B): scpBNA (C(B) represents 5-methylcytosine scpBNA); Upper case letter (M): 2'-OMe RNA; ^: phosphorothioate bond (PS bond); Chol: cholesterol | | | |

The first nucleic acid strands described above target SR-B 1 gene and are composed of 14mer gapmer having a base sequence complementary to a part of the SR-B1 mRNA. The first nucleic acid strand of Chol-HDO (LNA) consists of a 5'-wing region composed of 2 LNA nucleosides at the 5' end, a 3'-wing region composed of 2 LNA nucleosides at the 3' end, and 10 DNA nucleosides between them. In Chol-HDO (scpBNA), the whole of LNA nucleosides in the 5' and 3'-wing regions are substituted with scpBNA nucleosides.

On the other hand, the second nucleic acid strands are composed of a cholesterol-bound complementary strand RNA having a sequence complementary to the first nucleic acid strand and cholesterol bound at the 5' end thereof.

The double-stranded nucleic acid complexes were prepared by the same method as in Example 1 (1).

### (2) In vivo experiment

As mice to which a double-stranded nucleic acid complex agent was to be administered, 6 to 7 week-old male C57BL/6 mice having a weight of 20 g were used. For each condition, n=4 experiments were conducted.

The double-stranded nucleic acid complex agent was intravenously injected multiple times (administered once per week, 4 times in total) through the tail vein at a dose of 50 mg/kg. Further, PBS alone was injected in a single dose to mice as a negative control group.

At the time point of 72 hours after administration, blood was sampled and PBS was perfused in the mice. Thereafter, the mice were dissected and individual sites of the brain and whole body were isolated.

### (3) Expression analysis

Using a high-throughput fully automated nucleic acid extractor MagNA Pure 96 (Roche Life Science), mRNA was extracted from individual tissues in accordance with the protocol. In accordance with the protocol of Transcriptor Universal cDNA Master (Roche Life Science), cDNA was synthesized. Quantitative RT-PCR was performed using TaqMan (Roche Life Science). As the primers used in the quantitative RT-PCR, a product designed and produced by Thermo Fisher Scientific based on various number of genes, was used. The PCR conditions (temperature and time) were as follows: a cycle consisting of 95°C for 15 seconds, 60°C for 30 seconds, and 72°C for one second was repeated 40 times. Amplified products obtained were quantified by quantitative RT-PCR and the expression levels of SR-B 1 mRNA and Actin mRNA (internal standard gene) were calculated. The ratio of the expression levels of the two mRNAs was calculated to obtain a relative expression level. Mean values and standard errors of the relative expression levels were calculated. The differences between individual groups were analyzed by t-test.

### (4) Serum analysis

The serum obtained by sampling blood were analyzed by outsourcing to SRL Hachioji Lab.

### (Results)

The results of the expression analysis are shown in Figure 18. Chol-HDO (scpBNA) exhibited strong gene suppression effects in the brain (Figure 18A) and organs in the whole body (Figure 18B).

The results of the serum analysis are shown in Figure 19. After initial administration, AST/ALT significantly increased after 3 days in the Chol-HDO (LNA) administration group, and the mice died after 5 to 6 days. In contrast, in the Chol-HDO (scpBNA) administration group, mice were still alive ever after the 4th administration and the increase of AST/ALT was small.

From the results described above, it was demonstrated that the toxicity of HDO is reduced by placing scpBNA in the wing regions of a first nucleic acid strand, also in the case of multiple-dose administration.

### <Example 6: Effects of reduction of blood inflammatory cytokine/chemokine expression by single-dose intravenous administration of Toc-HDO (scpBNA)>

### (Purpose)

Blood inflammatory cytokine/chemokine expression are investigated when a double-stranded nucleic acid complex consisting of a scpBNA/DNA gapmer antisense nucleic acid and a tocopherol-bound complementary strand is intravenously administered.

### (Method)

### (1) Preparation of nucleic acid

In this Example, Toc-HDO (LNA), Toc-HDO (scpBNA), and Toc-HDO (AmNA) which target mMalat1, as listed in Table 3; Toc-HDO (LNA), Toc-HDO (scpBNA), and Toc-HDO (AmNA) which target PTEN, as listed in Table 4; and Toc-HDO (LNA), Toc-HDO (scpBNA), and Toc-HDO (AmNA) which target SR-B1, as listed in Table 5, were used. The double-stranded nucleic acid complexes were prepared by the same method as in Example 1 (1).

### (2) In vivo experiment

*In vivo* experiment was performed in the same manner as in Example 2 except that the dose of HDO targeting mMalat1 was 50 mg/kg and the dose of HDO targeting PTEN was 35 mg/kg. The double-stranded nucleic acid complex agent was intravenously administered in a single dose through the tail vein. At the time point of 72 hours after administration, blood was sampled.

### (3) Quantification of inflammatory cytokine/chemokine in the blood

Blood obtained by sampling was analyzed using MILLIPLEX MAP Mouse Cytokine/Chemokine Magnetic Bead Panel-Immunology Multiplex (Millipore) in accordance with the method disclosed in the instructions. To evaluate toxicity by a nucleic acid agent, the following protein group: G-CSF, GM-CSF, IFN-γ, IL-1α, IL-1β, IL-2, IL-3, IL-4, IL-5, IL-6, IL-7, IL-9, IL-10, IL-12 (p40), IL-12 (p70), IL-13, IL-15, IL-17, IP-10, KC, LIF, LIX, MCP-1, M-CSF, MIG, MIP-1α, MIP-1β, MIP-2, RANTES, TNFα, VEGF, and Eotaxin/CCL11 were subjected to the measurement of the amount of protein. As a result, TNFα, IP-10, and RANTES which increased by administration of Toc-HDO (LNA) were measured as indice of inflammation.

### (Results)

The measurement results of inflammatory cytokine levels in the blood are shown in Figure 20 to Figure 22.

As shown in Figure 20 to Figure 22, it was demonstrated that the expression levels of all of TNFα (Figure 20), IP-10 (Figure 21), and RANTES (Figure 22) were low in the Toc-HDO (scpBNA) administration group and Toc-HDO (AmNA) administration group, compared to the Toc-HDO (LNA) administration group.

All publications, patents, and patent applications cited herein are incorporated herein in their entirety by reference.

## Claims

1. A double-stranded nucleic acid complex comprising a first nucleic acid strand and a second nucleic acid strand, wherein:
said first nucleic acid strand is capable of hybridizing to at least part of a target gene or a transcription product thereof, and has an antisense effect on said target gene or transcription product thereof,
said second nucleic acid strand comprises a base sequence complementary to said first nucleic acid strand, and
said first nucleic acid strand and/or said second nucleic acid strand comprises at least one bridged non-natural nucleoside represented by the following formula (I) or formula (II): 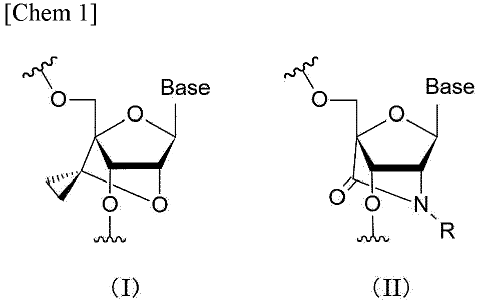
wherein R represents a hydrogen atom or a methyl group.

2. The double-stranded nucleic acid complex of claim 1, wherein said first nucleic acid strand is a gapmer.

3. The double-stranded nucleic acid complex of claim 2, wherein said first nucleic acid strand comprises:
(1) a central region which comprises at least four consecutive deoxyribonucleosides,
(2) a 5'-wing region which comprises a non-natural nucleoside, positioned on the 5' end side of said central region, and
(3) a 3'-wing region which comprises a non-natural nucleoside, positioned on the 3' end side of said central region.

4. The double-stranded nucleic acid complex of claim 3, wherein said 5'-wing region and/or said 3'-wing region comprises the bridged non-natural nucleoside represented by said formula (I) or formula (II).

5. The double-stranded nucleic acid complex of claim 3 or 4, wherein said second nucleic acid strand comprises at least four consecutive ribonucleosides which are complementary to the at least four consecutive deoxyribonucleosides in said central region in said first nucleic acid strand.

6. The double-stranded nucleic acid complex of claim 5, wherein said second nucleic acid strand further comprises at least two consecutive deoxyribonucleosides.

7. The double-stranded nucleic acid complex of any one of claims 3 to 6, wherein said second nucleic acid strand comprises the bridged non-natural nucleoside represented by said formula (I) or formula (II) in a region which consists of a base sequence complementary to the 5'-wing region and/or the 3'-wing region in said first nucleic acid strand.

8. The double-stranded nucleic acid complex of claim 1, wherein said first nucleic acid strand is a mixmer.

9. The double-stranded nucleic acid complex of any one of claims 1 to 8, wherein said first nucleic acid strand and/or said second nucleic acid strand comprises at least one nucleoside modified at the 2'-position of the ribose selected from the group consisting of 2'-O-methyl modified nucleoside, 2'-O-methoxyethyl modified nucleoside, and 2'-O-[2-(N-methylcarbamoyl)ethyl] modified nucleoside.

10. The double-stranded nucleic acid complex of any one of claims 1 to 9, wherein all or part of the internucleoside bond in said first nucleic acid strand and/or said second nucleic acid strand are a modified internucleoside bond.

11. The double-stranded nucleic acid complex of claim 10, wherein said modified internucleoside bond is a phosphorothioate bond.

12. The double-stranded nucleic acid complex of any one of claims 1 to 11, wherein said second nucleic acid strand is bound to a tocopherol or an analog thereof, or a cholesterol or an analog thereof.

13. The double-stranded nucleic acid complex of any one of claims 1 to 11 which is not bound to a ligand.

14. The double-stranded nucleic acid complex of any one of claims 1 to 13, wherein said first nucleic acid strand and said second nucleic acid strand are bound via a cleavable or uncleavable linker.

15. A pharmaceutical composition comprising the double-stranded nucleic acid complex of any one of claims 1 to 14 as an active ingredient.

16. The pharmaceutical composition of claim 15 for treating a central nervous system disease in a subject.

17. The pharmaceutical composition of claim 15 or 16, wherein the pharmaceutical composition is administered by intraventricular administration or intrathecal administration.

18. The pharmaceutical composition of claim 17, wherein 0.1 mg to 200 mg of said double-stranded nucleic acid complex is administered.

19. The pharmaceutical composition of claim 15 or 16, wherein the pharmaceutical composition is administered by intravenous administration or subcutaneous administration.

20. The pharmaceutical composition of claim 19, wherein said double-stranded nucleic acid complex is administered at 0.1 mg/kg to 100 mg/kg.

21. The pharmaceutical composition of any one of claims 15 to 20, wherein induction of inflammation or gliosis, or abnormal increase of a cytokine or a chemokine by the pharmaceutical composition is reduced.
